# EUROPEAN PATENT SPECIFICATION

(11) **EP 2 838 436 B1**
(45) Date of publication and mention of the grant of the patent: **13.03.2019**
(21) Application number: 13721833.5
(22) Date of filing: 18.04.2013
(51) Int. Cl.: A61B 17/02, A61B 17/34

(54) **NATURAL ORIFICE SURGERY SYSTEM**
CHIRURGISCHES SYSTEM FÜR NATÜRLICHE ÖFFNUNG
SYSTÈME CHIRURGICAL POUR ORIFICE NATUREL

(30) Priority: 20.04.2012 US 201261636492 P
(43) Date of publication of application: 25.02.2015
(60) Divisional application: 19151981.8
(73) Proprietor: Applied Medical Resources Corporation, Rancho Santa Margarita, CA 92688 (US)
(72) Inventor: DANG, Kevin K., Garden Grove, CA 92845 (US); ALBRECHT, Jeremy J., Rancho Santa Margarita, CA 92688 (US); BROWN, Blaze, Rancho Santa Margarita, CA 92688 (US); HOKE, Adam, Columbia City, IN 46725 (US); SAIDUDDIN, Adeeb, Irvine, CA 92620 (US); JOHNSON, Gary, Rancho Santa Margarita, CA 92688 (US); FILEK, Jacob J., Rancho Santa Margarita, CA 92688 (US)
(74) Representative: Dolleymores
(86) International application number: PCT/US2013/037213
(87) International publication number: WO 2013/158906

(56) References cited:
- EP-A2- 2 272 450
- US-A- 4 117 847
- US-A- 6 033 428
- US-A1- 2012 095 297

## Description

### BACKGROUND

### Technical Field

This application is generally directed to surgical devices, and more particularly, to a retractor adapted for use with a cap, that is useful in natural orifice single-port surgical procedures.

### Description of the Related Art

Access devices are commonly used in surgery to facilitate the introduction of various surgical instruments into natural biological vessels, conduits, orifices, cavities, and other interior regions of the body. These access devices include, for example, devices that facilitate the introduction of a needle into a vessel, and trocars that facilitate the introduction of laparoscopic instruments into the abdomen of the body.

Some of these access devices are introduced into regions that include a fluid or gas under pressure. In the case of a needle access device, the pressure may be from a liquid, such as blood. In the case of a trocar, the pressure may be from a gas, such as an insufflation gas. In either case, it is desirable to provide for the introduction of the surgical instrument into the cavity without permitting the escape of the pressurized fluid or gas.

In the case of trocars, a cannula at the distal end of the trocar is typically connected to a seal housing at the proximal end of the trocar. Together the cannula and housing form a working channel through which various instruments can be inserted to access the cavity. Seal mechanisms are commonly disposed in the housing and include a septum valve that seals the working channel when an instrument is in place, and a zero closure valve that seals the working channel when the instrument is removed.

Current surgical access ports allow for single instrument access through each port, or allow for multiple instrument access through a rigid cannula. Some devices, such as transanal endoscopic microsurgery (TEMS) units require that the device be attached to the surgical table to support the weight of the device, as well as to locate the position of the device respective to the patient. These devices do not provide flexibility to the surgeon in selecting instrument size, and they restrict instrument movement with their rigid cannulas. Additionally, surgeons are performing laparoscopic surgical procedures through a single or a limited number of access ports. The procedures may be performed through a single two (2) centimeter incision at the umbilicus, or in certain cases, trans-vaginally or trans-anally. What is needed is a system that meets the needs of these new procedures, facilitating more flexible movement of laparoscopic instruments through a single or limited number of ports while preventing the escape of pressured fluids or gasses and permitting large specimen removal.

US2012/095297 A1 discloses a surgical access port system comprising a retractor sized to fit within a natural orifice with minimal distention of the orifice. The two-part form of independent claim 1 is based on this document.

EP 2 272 450 A2 discloses an access port for performing transvaginal surgery comprising a retention mechanism to secure the distal end of the access port within the abdominal cavity of the patient.

US 4 117 847 A discloses a colon catheter suitable for the introduction of enema fluid into the patient after the catheter has been introduced into the patient.

### SUMMARY OF THE INVENTION

The invention is directed to a surgical access port, as defined in appended claim 1, that comprises a retractor that is adapted for being coupled to a cap and that is particularly useful in natural orifice surgery. Preferred embodiments are defined in the appended dependent claims.

The retractor comprises an outer ring, wherein the outer ring is configured to be disposed proximate the natural orifice of the patient and substantially surround the orifice; a tubular body; a funnel segment extending between and coupling the outer ring and the tubular body, wherein the funnel segment provides a diametric reduction between the relatively large diameter of the outer ring and the relatively smaller diameter of the tubular body, which is sized to fit within a natural orifice with minimal distention of the orifice; and an inflatable member disposed around the distal end of the tubular body, the inflatable member sized and configured to fit snugly around the tubular body in the deflated condition and to expand against the wall of the natural orifice in the inflated state to thereby stabilize and retain the retractor within the orifice. The surgical access port system further comprises an obturator comprising a proximal bearing surface sized and configured to bear against an inner surface of the funnel segment, a distal dilation surface sized and configured to expand the natural orifice, and an indent disposed along the periphery of the proximal bearing surface sized and configured to provide clearance for the inflation port.

In one aspect, the tubular body comprises a substantially flexible material, such as a KRATON® material, a PELLETHANE® material or a silicone rubber material. In another aspect, the tubular body comprises a more rigid material, such as polycarbonate. The tubular body defines a generally cylindrical passage large enough to accommodate at least one laparoscopic instrument there through, and preferably is sufficiently large such that two or more surgical instruments positioned there through can be translated or pivoted relative to one another. In one aspect, the tubular body comprises one or more coatings, such as an antimicrobial coating. In one aspect, the tubular body has an adjustable length, where, for example, it comprises interlocking sections or perforations. In another aspect, the tubular body has opening or windows along the length of the body, to provide access by surgical instruments to the body cavity or orifice.

In one aspect, the funnel segment comprises an inner surface that can provide a bearing surface for an obturator used to advance to the retractor into a body cavity. The funnel segment can have a substantially linear taper between the relatively large diameter of the outer ring and the relatively smaller diameter of the tubular body. In one aspect, the funnel segment has a curved profile between the relatively large diameter and the relatively smaller diameter.

In one aspect, the tubular body can be formed from a relatively flexible material and the funnel segment and the outer ring from a relatively rigid material. The surgical access port system can further comprise an obturator.

In one aspect, the surgical access port system further comprises a removable cap, wherein the cap is adapted to sealingly engage the outer ring. In one aspect, the cap has a sealable access surface, such as a gel pad, and can include at least one fluid or gas port. In one aspect, the surgical access port system further comprises at least one trocar access device, wherein the trocar access device is adapted to be positioned through the sealable access surface. The trocar access device preferably contains at least one sealing valve, such as a septum seal or duck bill valve. In one aspect, the trocar access device has a low profile.

These and other features and advantages of the invention will become more apparent with a discussion of embodiments in reference to the associated drawings.

### BRIEF DESCRIPTION OF THE DRAWINGS

FIG. **1** is a side view of a patient in surgery illustrating an embodiment of the access device positioned on the abdomen and in use.
FIG. **2** is a cross-sectional side view illustrating an embodiment of the access device, with the wound retractor retracting the vagina of a patient, and the gel cap sealing the opening of the wound retractor.
FIG. **3** is a front view illustrating an embodiment of the access device deployed and in use at the mouth of the patient.
FIG. **4** is a top view illustrated a patient in the prone position with an embodiment of the access device deployed and in use at the anus of the patient.
FIG. **5** is a perspective view of an access device comprising a cap and a retractor.
FIG. **6A** is a side view of a natural orifice retractor. FIG. **6B** is a top view of the natural orifice retractor of FIG. **6A****.** FIG. **6C** is a partial cut away of the natural orifice retractor of FIG. **6A****.**
FIG. **6D** is a side view of another natural orifice retractor. FIG. **6E** is a top view of the natural orifice retractor of FIG. **6D****.** FIG. **6F** is a perspective view of the natural orifice retractor of FIG. **6A**.
FIG. **6G** is a perspective view of an obturator adapted to facilitate introduction of a natural orifice retractor into a body orifice such as an anus. FIG. **6H** is a side view of the obturator of FIG. **6G**.
FIG. **6I** is a perspective view of an obturator having a straight shaft piece, adapted to facilitate introduction of a natural orifice retractor into a body orifice such as an anus. FIG. **6J** is a perspective view of a retractor disposed on the obturator of FIG. **6I**.
FIG. **7A** is a partial side cross section of the natural orifice retractor of FIG. 6A with a gel cap coupled therewith to form a natural orifice access device.
FIG. **7B** is a side cross section of the natural orifice retractor of FIG. **6D**.
FIG. **7C** is a perspective view of a natural orifice retractor formed from sections and having cut-out portions or windows in the tubular body of the retractor. FIG. **7D** is a cutaway view of the retractor of FIG. **7C** showing the slidable engagement of the sections. FIG. **7E** is a cutaway view of the retractor of FIG. 7C showing the snap-lock mechanism securing the sections together.
FIG. **7F** is a perspective view and a side view of a retractor having cut-out portions or windows in the tubular body of the retractor.
FIG. **7G** is a perspective view of an embodiment of a retractor according to the present invention having an inflatable member. FIG. **7H** shows a close-up view of the inflatable member. FIG. **7I** is a top-down perspective view of a retractor showing the check valve port of inflating the inflatable member. FIG. **7J** is a cutaway side view showing the check valve and channel disposed in the tubular body of the retractor. FIG. **7K** is a side view of a retractor showing the channel disposed between the check valve and the inflatable member.
FIG. **7L** is a perspective view of an obturator, modified with an indent to provide clearance for the inflation port shown in FIG. **7J** and **7K** and adapted to facilitate introduction of a natural orifice retractor into a body orifice such as an anus.
FIG. **7M** is a perspective view and a side view of a perforated natural orifice retractor.
FIG. **8A** is a side view of the natural orifice access device of FIG. **7A**. FIG. 8B is a top view of the natural orifice access device illustrated in FIG. **7A**. FIG. **8C** is a perspective view of the natural orifice access device illustrated in FIG. **7A**.
FIG. **8D** is a perspective view of the natural orifice retractor of FIG. **6D** with a gel cap therewith to form one embodiment of natural orifice access device.
FIG. **9A** is a perspective view of a natural orifice access device including a cap having a plurality of trocars extending there through. FIG. **9B** is a perspective view of another natural orifice access device including a cap having a plurality of trocars extending there through.
FIG. **9C** is an exploded view of a trocar access device and obturator.

Similar components have similar reference numbers throughout.

### DETAILED DESCRIPTION OF CERTAIN EMBODIMENTS

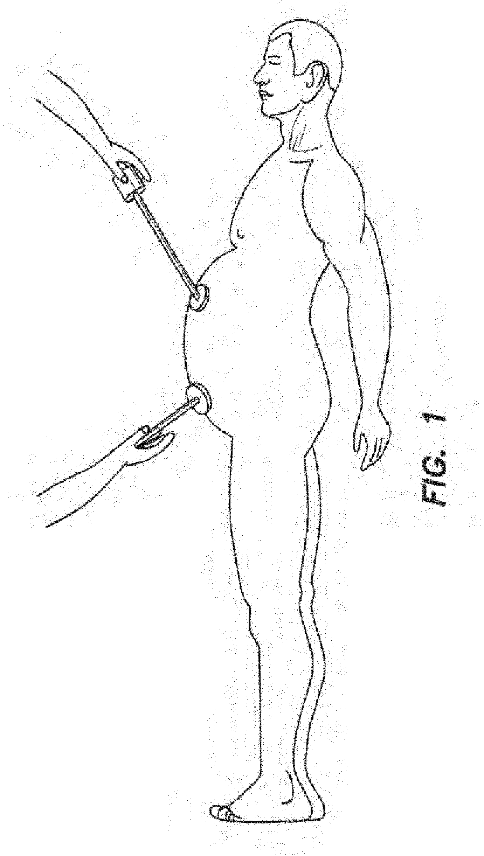

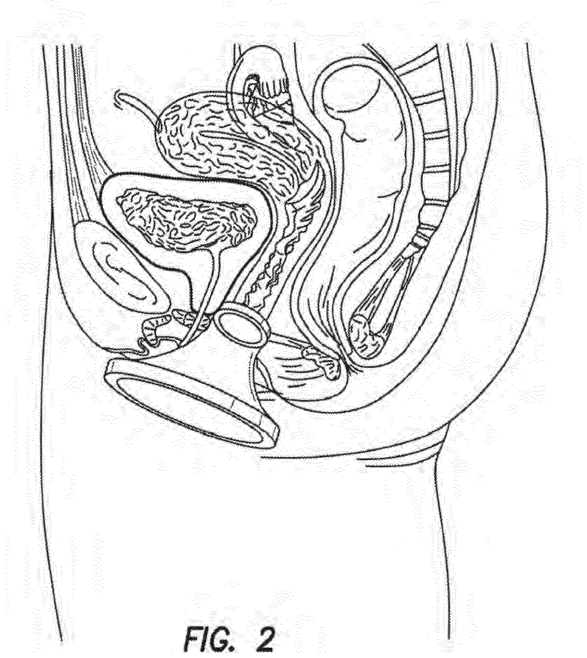

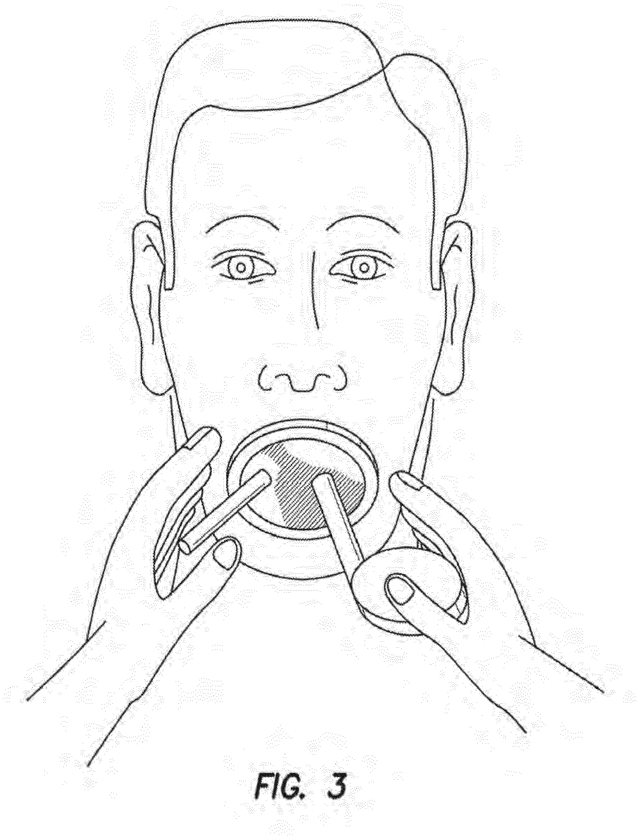

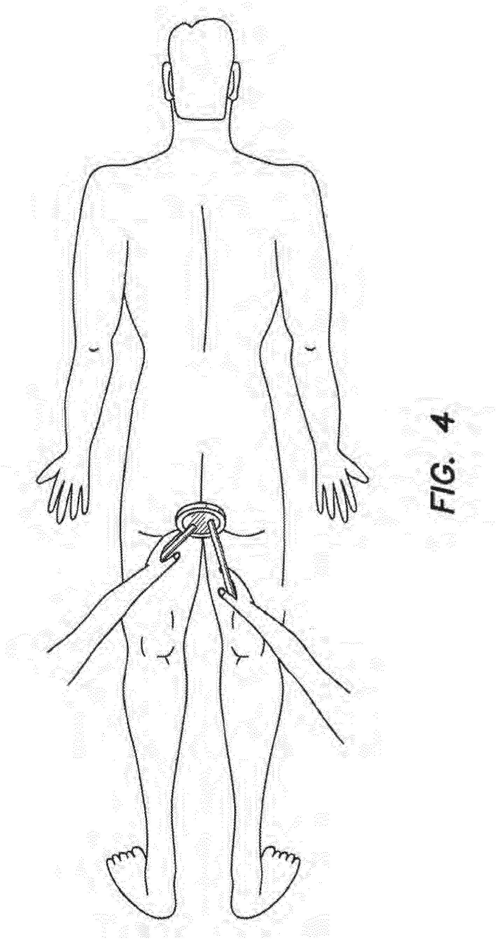

Embodiments of a surgical instrument access device system are useful, for example, for single incision, single port, and/or limited port laparoscopic surgical procedures, for example, abdominal (FIG. **1**), transvaginal (FIG. **2**), transoral (FIG. **3**), and transanal (FIG. **4**) procedures. Various surgical instrument access devices are described in U.S. Patent Application Publication No. 2009/0187079, and U.S. Patent No. 7,727,146.

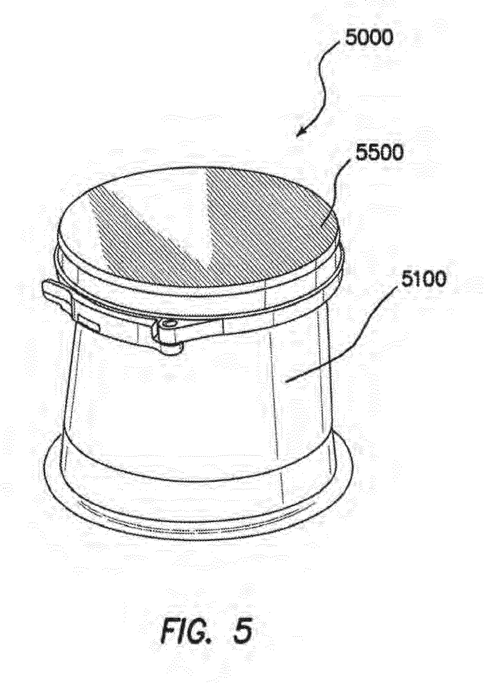

FIG. **5** illustrates a perspective view of an access device system **5000** comprising a retractor **5100** and a cap **5500**, which is useful in single port and/or limited port procedures. The retractor or surgical wound retractor **5100** is placed and/or positioned into, across, and/or through a surgical incision and/or body orifice to enlarge, reshape, and/or isolate the incision or body orifice. The cap **5500** provides an artificial body wall through which instruments access the interior of a patient's body, for example, a body cavity. The components of the access device **5000** comprise any suitable biologically compatible materials.

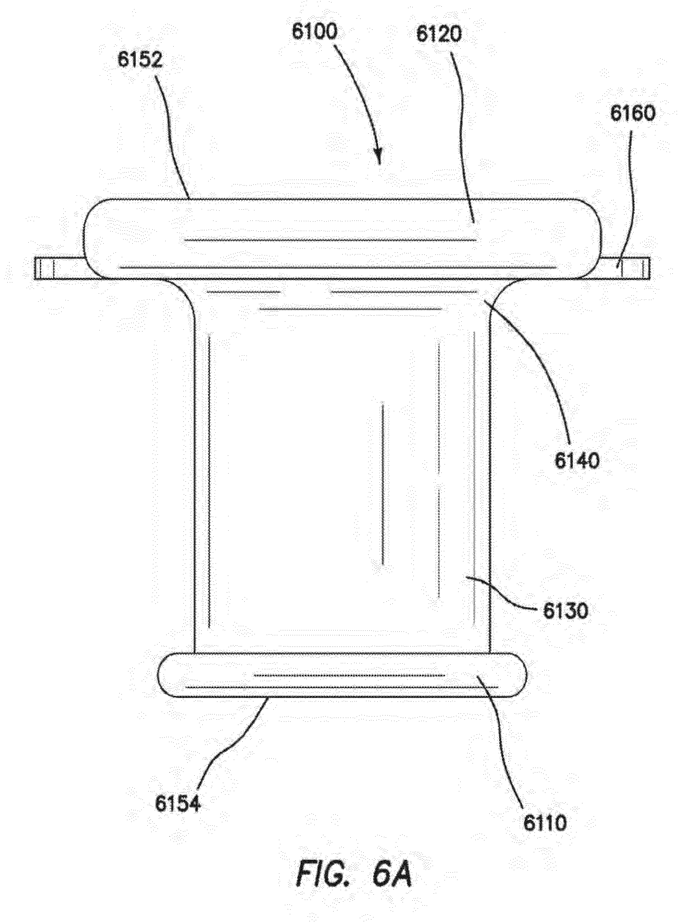

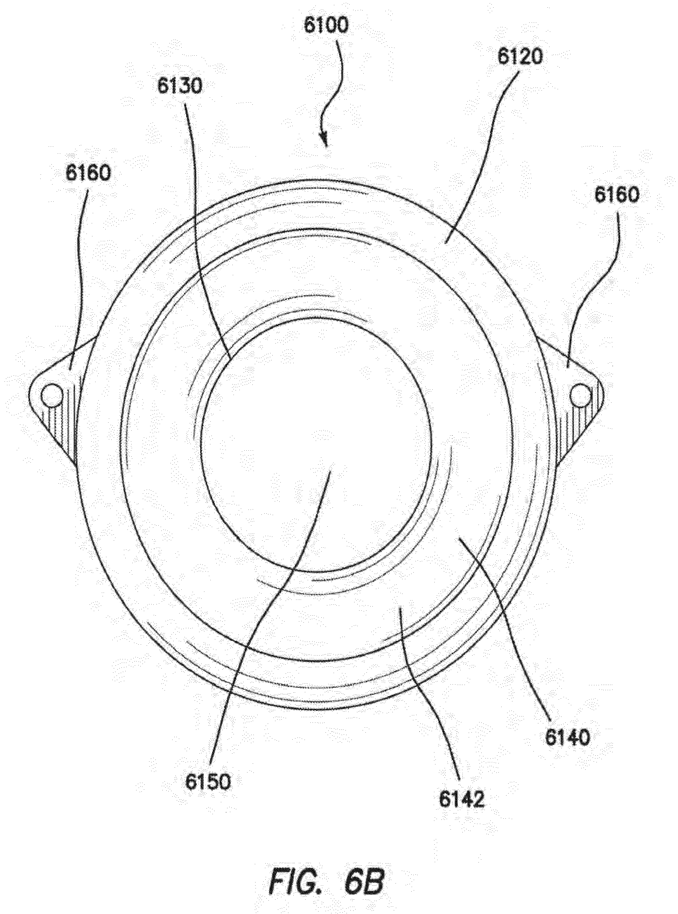

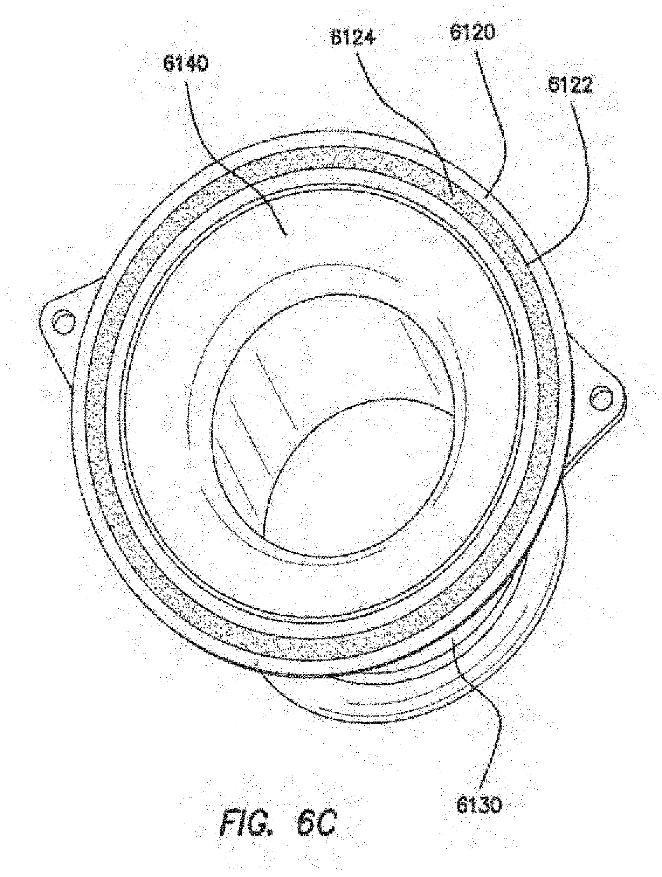

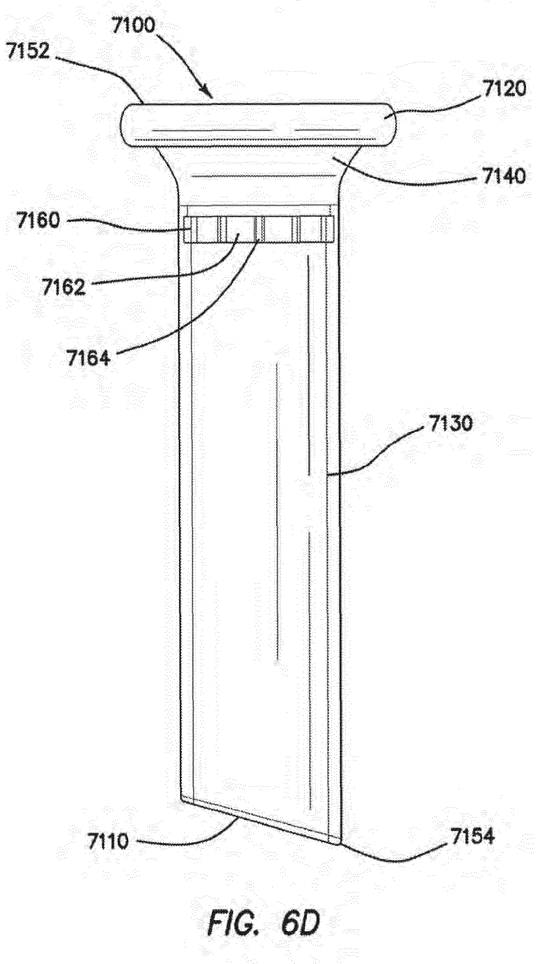

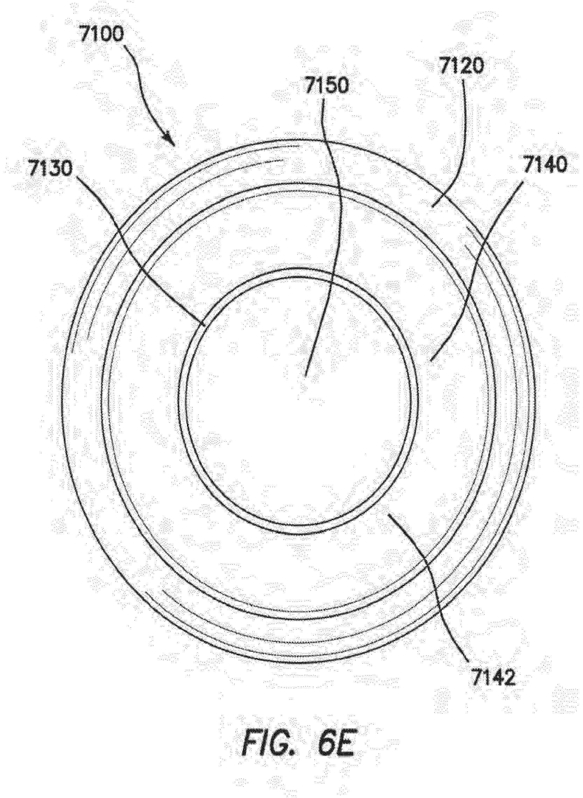

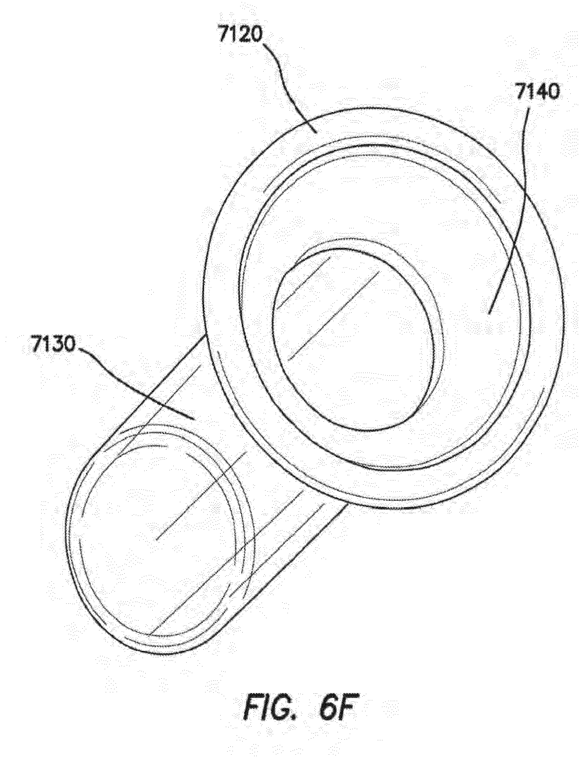

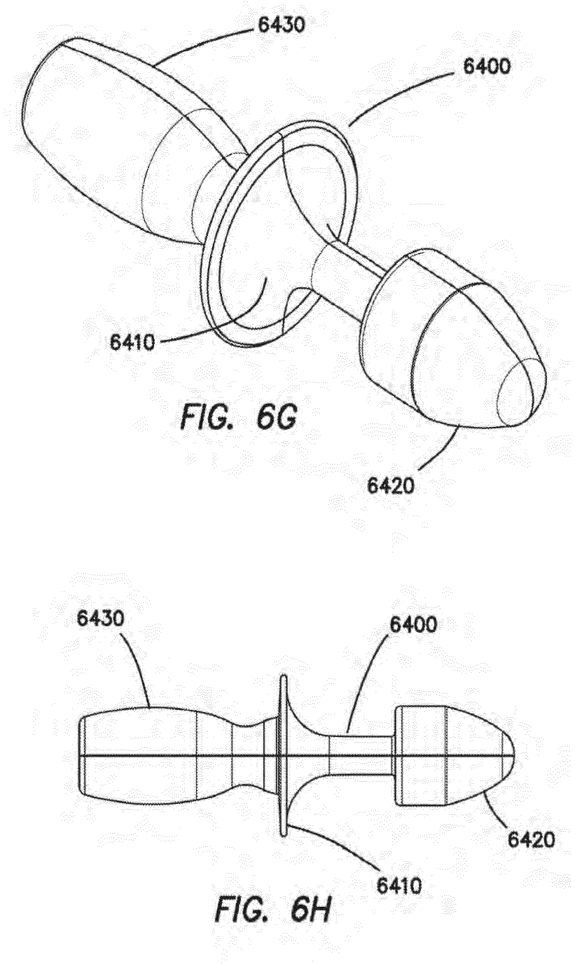

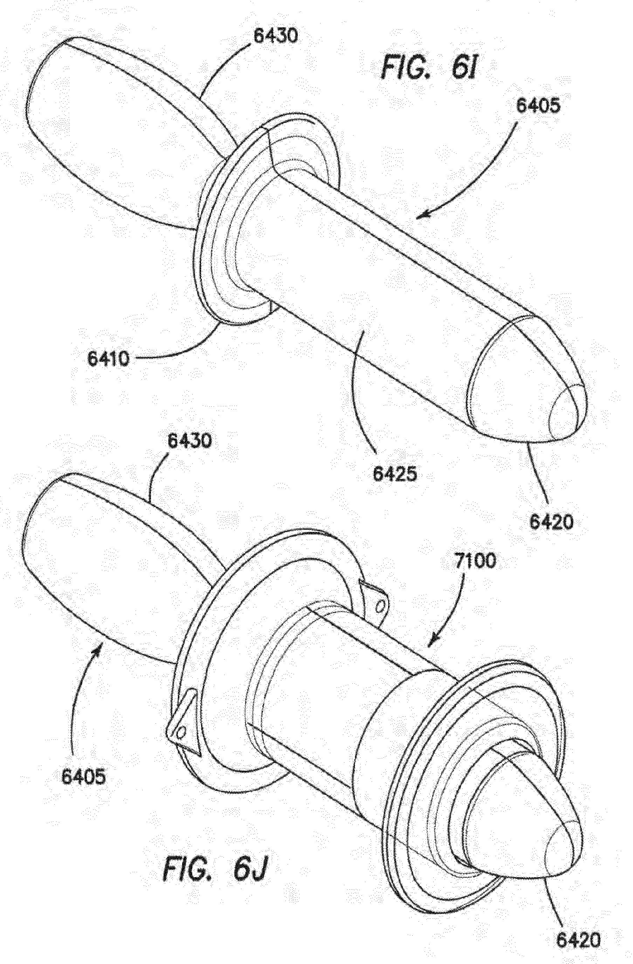

Two examples of natural orifice access ports or retractors **6100, 7100** sharing certain similarities are illustrated in FIGS. **6-9**. One example of retractor **6100** is illustrated in FIGS. **6A-6C****,** **7A****,** **8A-8C****,** and **9A****.** Another example of retractor **7100** is illustrated in FIGS. **6D-6F****,** **7B****,** **8D****.,** and **9B**

The example of the natural orifice access port or retractor **6100** illustrated in a side view in FIG. **6A** can be adapted for use in a transanal surgical procedure. The retractor **6100** comprises an inner or distal ring **6110,** an outer or proximal ring **6120,** a tubular body **6130,** and a funnel segment **6140** extending between and coupling the inner ring **6110** and the outer ring **6120.** The tubular body **6130** comprises a relatively flexible material such as a KRATON® material or a silicone rubber material, which is substantially cylindrical in the example shown. In other examples, the tubular body **6130** has another shape, for example, an oval cross section. Some embodiments of the tubular body **6130** comprise one or more coatings that provide additional functionality, for example, an anti-microbial coating.

Examples of the inner ring **6110** are sufficiently flexible and compliant to be compressed and/or deformed for insertion into a body orifice such as a patient's anus during a transanal surgical procedure. When subsequently released within an associated body cavity, the inner ring **6110** substantially returns to its original shape or footprint. In some examples of a retractor, the inner ring **6110** assumes a substantially circular shape in a relaxed state, for example, when released within a body cavity. In other examples, the inner ring **6110** has another shape in the relaxed state, for example, an oval. The inner ring **6110** assumes a different shape when compressed for insertion through an incision or body orifice, for example, a substantially oval shape, a generally linear shape, a tear-drop shape, or another suitable shape. In other examples, the inner ring **6110** in the relaxed state has a shape other than round, for example, oval, elliptical, or D-shaped. In other examples, the inner ring **6110** is substantially rigid, that is, non-compliant under the ordinary conditions under which it is used. In some examples, the inner ring extends outward from the surface of the tubular body, as shown, for example, in FIG. 6A, to thereby aid in retaining the retractor in the body cavity after it is deployed.

Examples of the inner ring **6110** can comprise a generally circular cross section. In other examples, the inner ring **6110** comprises another cross-sectional shape, for example, at least one of oval or elliptical, tear-drop shaped, and D-shaped. For example, in retractors illustrated in FIGS. **6D-6F****,** the inner ring **7110** has a cross-sectional shape that is substantially flush with the tubular body **7130** of the retractor **7100** as further described herein. Other cross sections are used in other examples of a retractor. As further discussed herein with respect to the flexion region of the inner ring **6110,** some examples of the inner ring **6110** comprise at least one notch and/or weak spot, which facilitate folding or deforming the inner ring **6110,** thereby facilitating insertion and/or removal of the inner ring **6110.**

Returning to FIG. **6A**, the outer ring **6120** is proximal the funnel section **6140.** In the retractor shown, the outer ring **6120** has a substantially circular footprint. As further discussed herein, the outer ring **6120** can be sized and configured to sealingly couple to a cap or other access device thereon. In some embodiments, one or more suture points **6160** can be disposed on the retractor **6110** adjacent the outer ring **6120.**

With reference to FIG. **6B**, a top view of retractor **6100** is illustrated. In the illustrated retractor, outer ring **6120** has a generally circular profile. Additionally, in the illustrated retractor, two suture points **6160** are generally diametrically opposed relative to the generally circular profile of the outer ring **6120.** In other examples, the retractor can include more or fewer than two suture points disposed of various locations relative to the outer ring **6120.**

With continued reference to FIG. **6B**, the tubular body **6130** has a generally circular profile defining a generally cylindrical passage **6150.** The generally cylindrical passage **6150** is desirably large enough to accommodate more than one laparoscopic instrument there through such that a single natural orifice access device can be used to provide access for multiple surgical instruments in a body cavity. Moreover, generally cylindrical passage **6150** is desirably large enough such that multiple surgical instruments positioned there through can be translated or pivoted relative to one another, allowing a surgeon to manipulate the instruments as desired during a surgical procedure. The generally cylindrical passage extends between a proximal end **6152** of the retractor **6100** adjacent the outer ring **6120** to a distal end **6154** of the retractor **6100** adjacent the inner ring **6110** (FIG. **6A**).

With continued reference to FIG. **6B**, in the illustrated retractor, the funnel segment **6140** provides a diametric reduction between the relatively large diameter of the outer ring **6120,** which is sized and configured to be removably coupled to an access device such as a cap, and the relatively smaller diameter of the passage **6150,** which is sized to fit within a natural orifice with minimal distention of the orifice. The funnel segment **6140** has an inner surface **6142** which can provide a bearing surface for an obturator used to advance to the retractor **6100** into a body cavity. In some examples, the funnel segment **6140** can have a substantially linear taper between the relatively large diameter and the relatively smaller diameter such that the inner surface **6142** is a frusto-conical segment. In other examples, the funnel segment **6140** can have a curved profile between the relatively large diameter and the relatively smaller diameter.

In some examples, a natural orifice access system includes a retractor **6100** and an obturator **6400** (FIG. **6G-6H**). The obturator can have a proximal bearing surface **6410** sized and configured to bear against the inner surface **6142** of the funnel segment **6140** and a distal dilation surface **6420** sized and configured to expand a natural orifice for passage of the retractor **6100.** Thus, during insertion of the retractor **6100** into a natural orifice, the dilation surface **6420** expands a pathway to a surgical site in a body cavity while the obturator bears on the inner surface **6142** of the funnel segment **6140** to advance the retractor **6100** into position in the surgical site. Furthermore, in some retractors, the obturator can have a handle **6430** at a proximal end thereof adapted to facilitate selective twisting or rotation of the obturator about a longitudinal axis thereof during insertion.

It can be desirable that the outer ring **6120** is relatively stiff compared with the relatively flexible tubular body **6130** of the retractor **6100** so that the outer ring **6120** can sealingly engage an access device such as a cap. With reference to FIG. **6C**, a perspective view of an example of a retractor is illustrated with a partial cutaway of the outer ring **6120.** In the illustrated example, the outer ring **6120** includes an annular groove **6122** formed therein in which a reinforcing member **6124** is disposed. In some retractors, the reinforcing member **6124** can comprise a metallic member such as a wire formed into a ring shape. For example, the reinforcing member **6124** can comprise a stainless steel ring positioned within the groove **6122** during manufacture of the retractor **6100**. In other examples, the reinforcing number **6124** can comprise an injectable nonmetallic member. For example, a glass filled polymer or polycarbonate material can be injected into the groove **6122** during manufacture of the retractor **6100**.

While the illustrated examples of retractor **6100** include a reinforcing member to enhance the rigidity of the outer ring **6120**, in other examples, the retractor **6100** can be formed in a multiple-shot molding process. For example, an inner segment of the retractor defined by the tubular body **6130** and the inner ring **6110** is formed in one molding operation from a flexible material, and an outer segment of the retractor **6100** defined by the funnel segment **6140** and the outer ring **6120** is formed in another molding operation from a relatively rigid material such as a polycarbonate material or other suitable material. One example of a retractor **7100** formed in a multiple-shot molding process is illustrated in FIGS. **6D-F****,** **7B****,** **8D****,** and **9B**.

With continued reference to FIG. **6C**, the illustrated retractor includes a continuous generally annular groove. In other examples, a plurality of noncontiguous recesses can each receive one of a plurality of reinforcing members. Moreover, in some examples, the outer ring can include two or more concentric generally annular grooves, which each receive a corresponding reinforcing member.

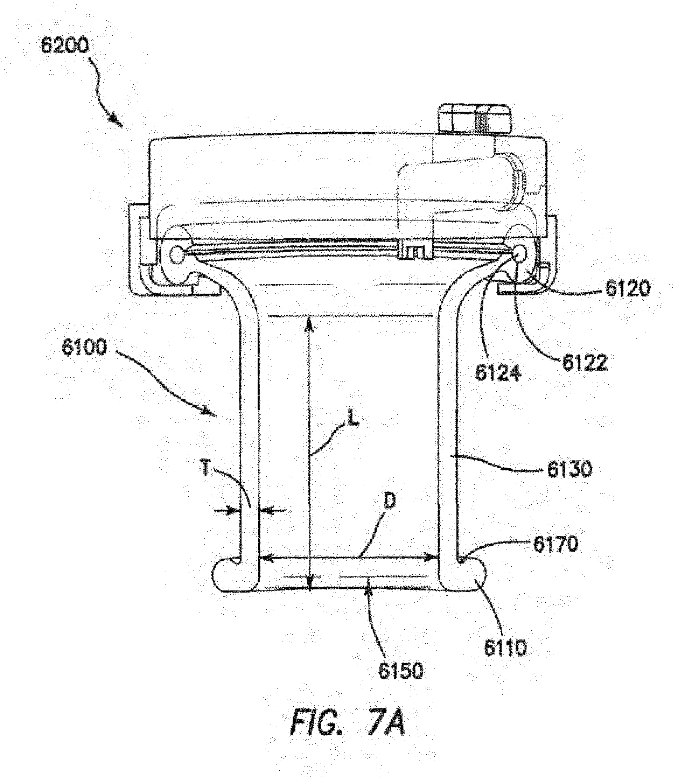

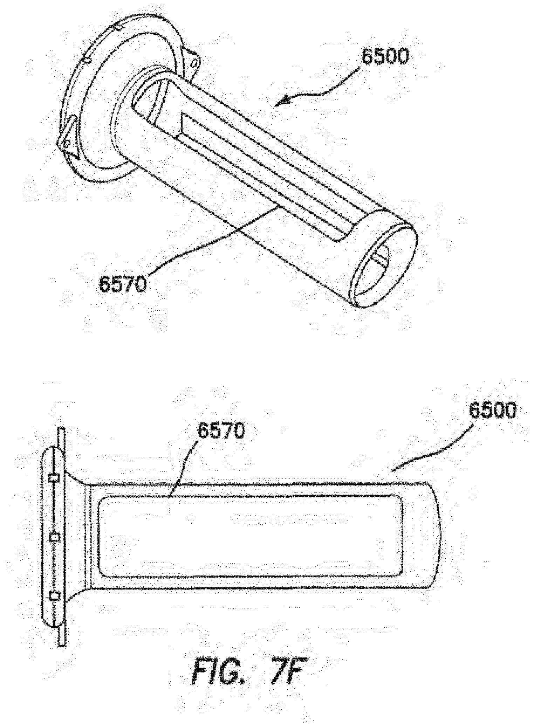

With reference to FIG. **7A**, a cross-sectional view of a natural orifice access device including a retractor **6100** and a removable cap **6200** is shown. In the illustrated example, the tubular body **6130** is formed of a flexible material having a predetermined fixed length L, inner diameter D, and wall thickness T. The fixed length L, inner diameter D, and wall thickness T are selected to accommodate the anatomy of a natural orifice, such as the anal orifice of a majority of patients. The retractor **6100** can be scaled to different sizes for patients of different ages. Furthermore, in some examples, the retractor can include a telescopic tubular body such that the tubular body can be selectively positioned at a variety of lengths depending on patient anatomy and the location of the surgical site within the body cavity. Desirably, the wall thickness T and material of the tubular body **6130** are selected such that the tubular body **6130** is resilient enough to maintain the passage **6150** there through when positioned in the natural orifice. Moreover, desirably, the inner diameter, D is sufficiently large to accommodate multiple surgical instruments. For example, in examples of the retractor **6100** adapted for use in a TEMS procedure, the inner diameter D and thickness T can be sized such that an outer diameter of the retractor can be between approximately 30 mm and 70 mm, desirably between approximately 35 mm and 50 mm, and in one example approximately 40 mm. Additionally, desirably, the fixed length L is sufficiently long such that the inner ring **6110** can be positioned at a surgical site within a body cavity and the outer ring **6120** can be positioned outside the natural orifice. In some retractors, the fixed length L is of a length such that the device has an overall length between the proximal end **6152** and the distal end **6154** of between approximately 10 mm and approximately 100 mm, desirably between approximately 20 mm and 80 mm, more desirably between approximately 30 mm and 60 mm, and in one embodiment, approximately 40 mm.

With continued reference to FIG. **7A**, in some retractors, the annular groove **6122** can be open to an inner surface of the outer ring **6120.** Thus, the retractor **6100** can be formed of a flexible material in a single molding operation with the annular groove **6122** having an opening, and the reinforcing member **6124** can be subsequently inserted into the upper groove **6122.**

With continued reference to FIG. **7A****,** in some examples, the retractor **6100** can include a flexion region between the tubular body **6130** and the inner ring **6110,** such as an undercut **6170.** Advantageously, the flexion region can allow the inner ring **6110** to flex or rotate relative to the tubular body **6130** during insertion such that the inner ring **6110** presents a relatively small outer diameter in an insertion configuration and a relatively larger outer diameter in an undisturbed configuration.

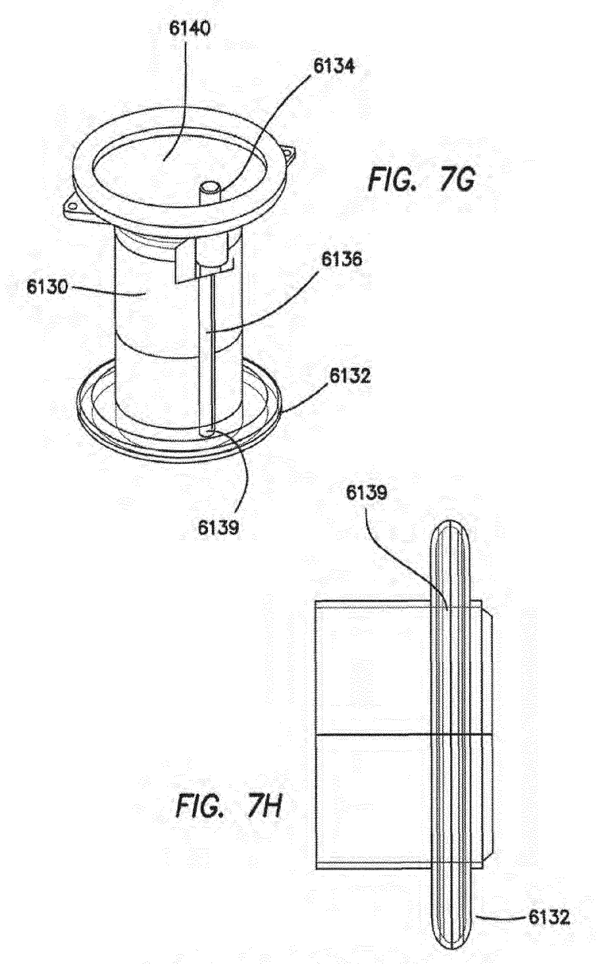

In embodiments of the surgical access port system according to the present invention, as shown in FIGS. **7G** - **7K**, the inner ring comprises an inflatable member **6132** such as an annular balloon coupled to a gas or fluid source that can be selectively inflated and deflated between a deflated, relatively small diameter state for insertion and removal, and an inflated, relatively high diameter state for retention in a body cavity. An inflation port **6134,** for example a check valve, affixed to the funnel portion **6140** of the retractor, is connected to the inflatable member **6132** through a channel **6136** within the wall of the tubular body **6130.** Fluid or gas introduced through the inflation port flows through the channel into the inflatable member to thereby inflate the member.

The channel **6136** runs through the tubular body, generally parallel to the longitudinal axis of the tubular body, with a proximal opening interacting with the inflation port **6134** and a distal opening **6139** into outer surface of the tubular body at the inflatable member. In one aspect, the inflation port **6134** may include a normally closed check valve having a spring-loaded plunger. In a further aspect, the check valve may include a Luer lock. It is contemplated that other inflation ports that are well known in the art may be used.

In this embodiment, the tubular body **6130** is preferably comprised of a relatively rigid material, such as a polycarbonate. The tubular body has an inflatable member at the distal end that may be created by heat shrinking polyolefin tubing around the outside of the tubular body. The distal end of the body/tubing assembly is then heated for approximately 30 to 40 seconds, and then placed inside a mold and injected with air to give the inflatable member an annular balloon shape as seen in FIG. **7H**, or any other desired shape, depending on the configuration of the mold. The inflatable member **6132** should have sufficient impermeability properties to substantially prevent inflation gas or fluid from permeating through a wall of the inflatable member.

In one embodiment, the inflatable member **6132** may include a substantially toroid shape upon inflation. In another embodiment, the inflatable member may include a disc shape upon inflation. In another embodiment, the inflatable member **6132** may be a fluted balloon. Other shapes suitable for particular natural orifices will be appreciated by one skilled in the art.

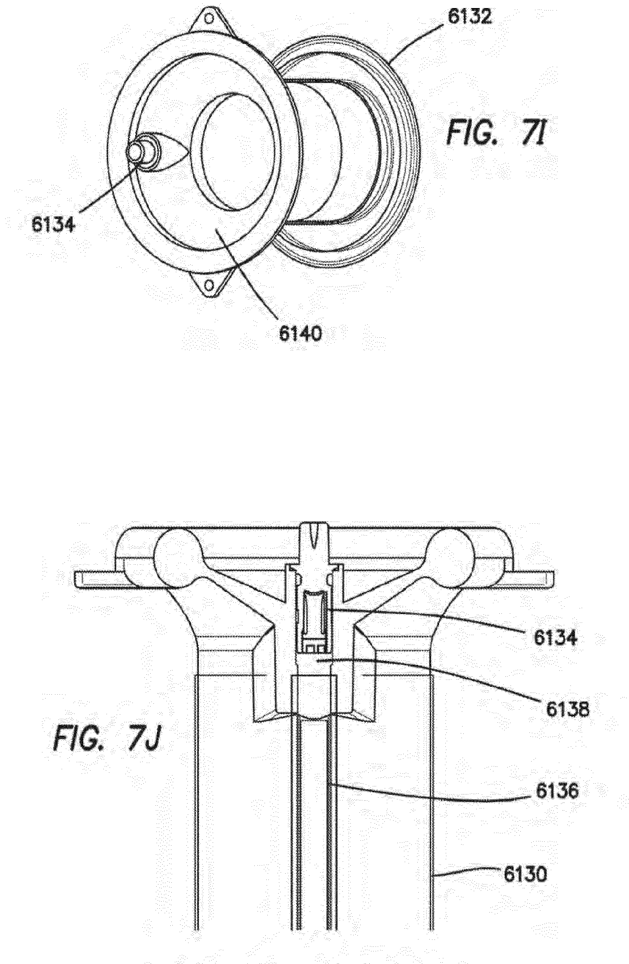

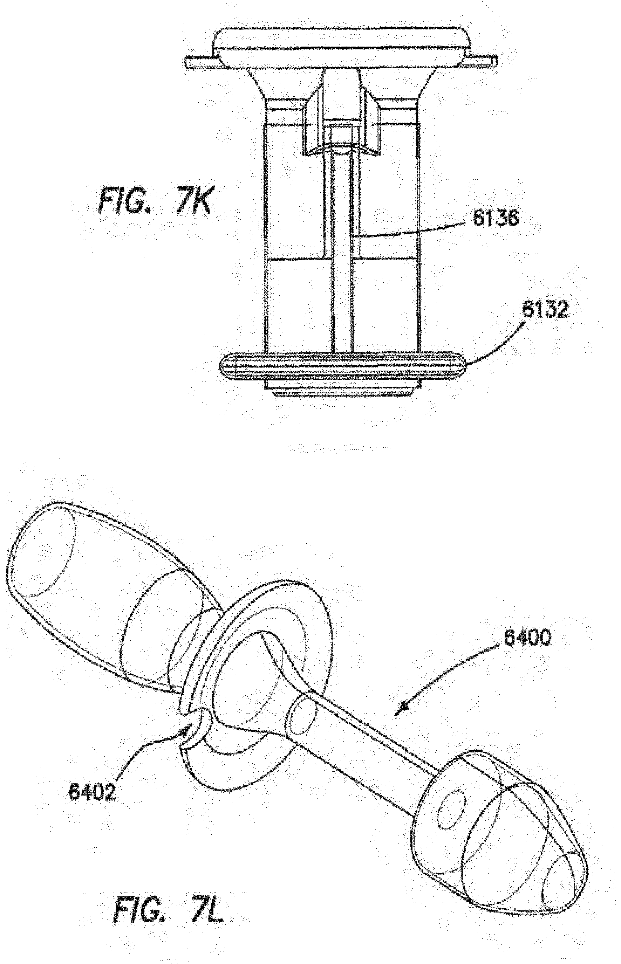

In use, the inflatable member may be inflated after the retractor is disposed within the natural orifice by inserting a syringe into the valve **6134** located at the proximal end **6138** of the channel within the tubular body (see FIG. **7I**). As shown in FIGs. **7J** and **7K**, the port leads into the channel **6136**, which allows the fluid or gas from the syringe to travel to the inflatable member **6132.** In this embodiment, the obturator **6400** is modified with an indent **6402** to provide clearance for the inflation port, as shown in FIG. **7L**.

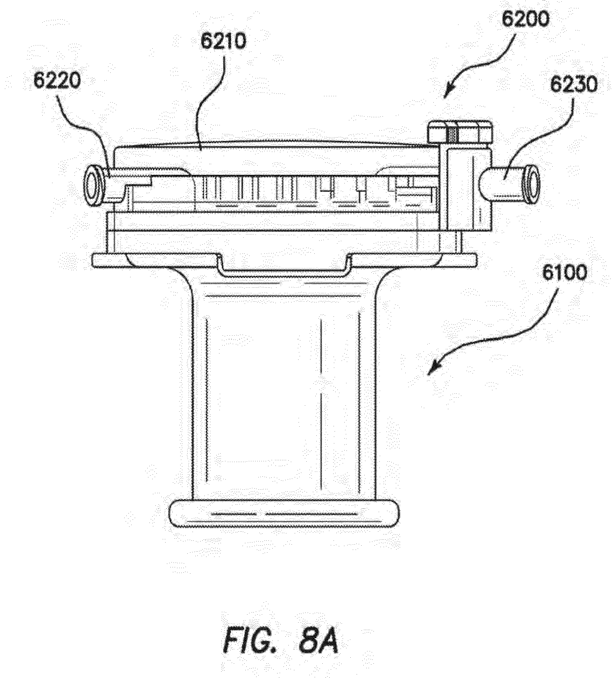

With reference to FIG. **8A**, a side view of a natural orifice access device having a cap **6200** removably coupled to a retractor **6100** is illustrated. In the illustrated device, the cap **6200** comprises a sealable access surface **6210** such as a gel pad surface as described in further detail herein. In certain devices, the cap **6200** can also comprise at least one gas or fluid port **6220, 6230.** In the illustrated device, the cap **6200** comprises two gas or fluid ports **6220, 6230,** such that one port can be used for gas insufflation and the other port can be used for ventilation for example when electrosurgery is performed through the access device. In certain devices, at least one of the gas or fluid ports **6220, 6230** comprises a valve such as a stopcock valve to selectively control the flow of fluid there through.

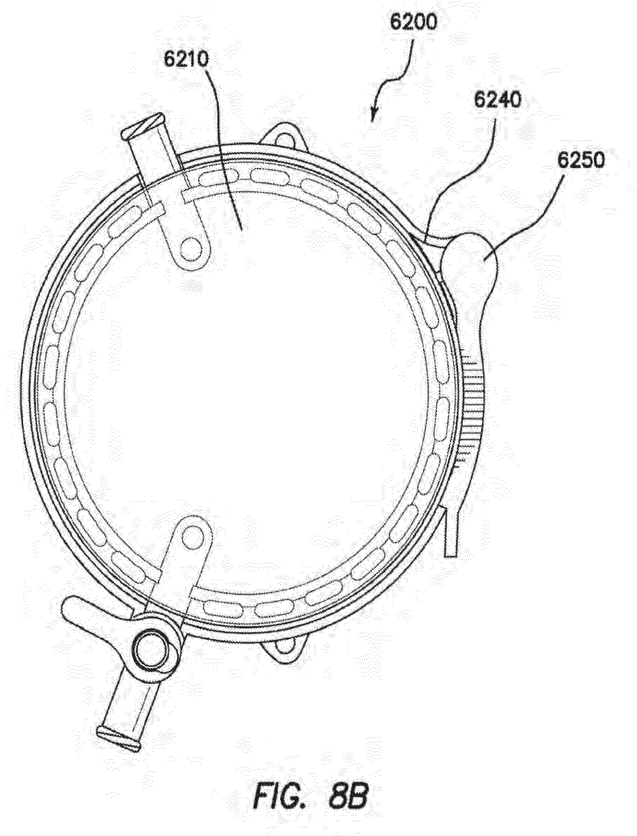

With reference to FIG. **8B**, a top view of the natural orifice access device is illustrated. The sealable access surface **6210** can be encircled by and restrained by an annular frame **6240** such as a split ring having a clamp **6250.** The clamp **6250** can be movable between an open configuration in which the cap **6200** is selectively removable from the retractor **6100** and a clamped configuration in which the cap **6200** can be secured to the retractor **6100.** For example, the annular frame **6240** can be positioned peripherally around the outer ring **6120** with the clamp **6250** in the open configuration and the clamp moved to the clamped configuration to sealingly fix the cap **6200** to the retractor **6100.** Accordingly, the cap **6200** can be easily removed during a surgical procedure to facilitate removal of excised tissue from a surgical site through the retractor **6100.**

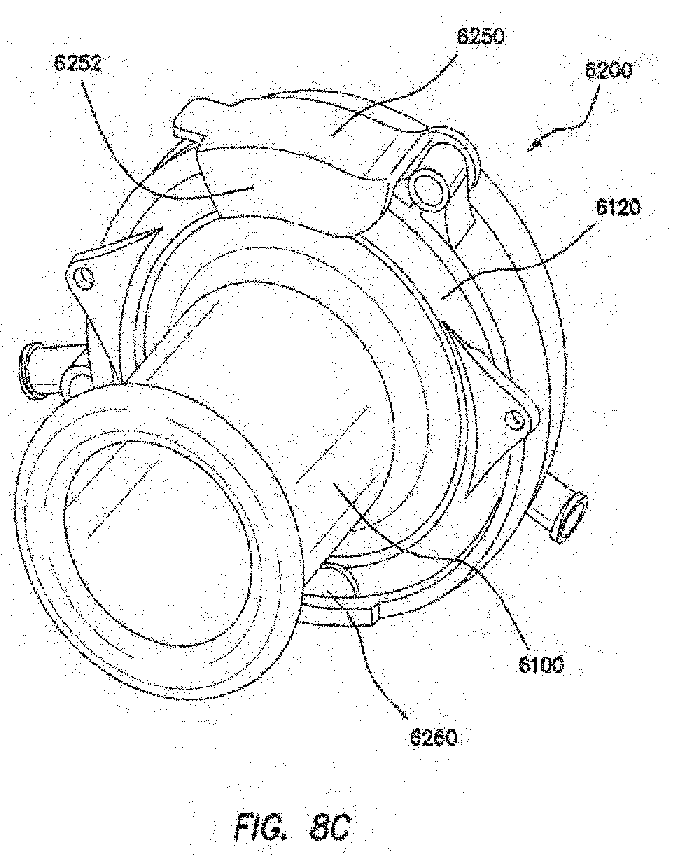

With reference to FIG. **8C**, a perspective view of a natural orifice access device is illustrated. In the illustrated device, the clamp **6250** can have a distal flange **6252** positioned to interface with the outer ring **6120** of the retractor when the clamp is in the clamped configuration. As illustrated, the clamp **6250** engages a distal surface of the outer ring **6120** of the retractor **6100.** In some device, the annular frame **6240** can further comprise at least one distal flange sized and positioned to interface with a retractor. In the illustrated device, the annular frame **6240** comprises a distal flange **6260** positioned to engage a distal surface of the outer ring **6120** of the retractor. As illustrated, the flange **6260** is generally diametrically opposed to the distal flange of the clamp **6250.** In other devices, the annular frame **6240** can include more than one distal flange positioned substantially equally spaced about the periphery of the annular frame **6240** or spaced irregularly about the periphery of the annular frame.

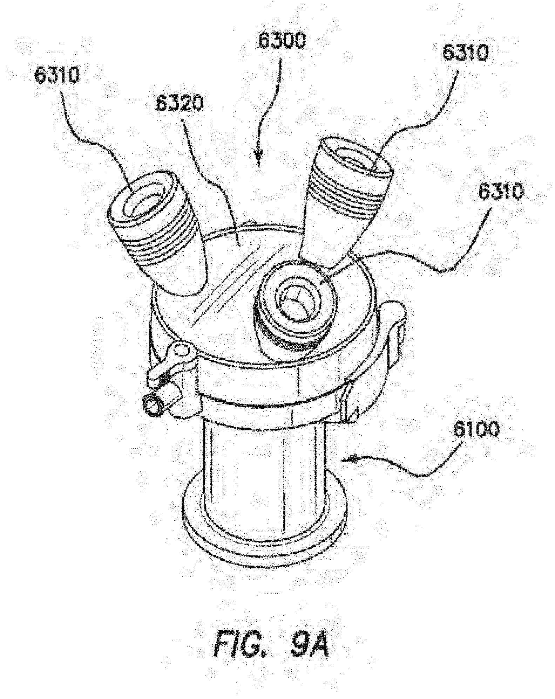

With reference to FIG. **9A****,** another example of a natural orifice access device is illustrated with a cap **6300** removably coupled to a retractor **6100** such as that described above with respect to FIGS. **6A-6C****,** **7A****,** **8A-8C****,** and **9A****.** In the illustrated device, the cap **6300** includes multiple trocar access devices **6310** positioned through an access surface **6320** thereof. Advantageously, the multiple trocar access devices **6310** allow for easy placement and manipulation of multiple laparoscopic instruments in a surgical site through a single natural orifice.

In some devices, the inner ring **6110** and the outer ring **6120** independently have different footprint shapes and/or footprint diameters. For example, in the retractor **7100** illustrated in FIGS. **6D-F****,** **7B****,** **8D****,** and **9B****,** the inner ring **7110** can be substantially flush with the tubular body **7130** while the outer ring **7120** can be an annular member having a generally circular cross-section. An inner ring **6110** with a larger diameter permits a greater retraction force, but is more difficult to insert and remove from a body cavity.

With reference to FIGS. **6D-6F****,** in some examples, a natural orifice access port or retractor **7100** can be adapted for use in a transanal endoscopic microsurgery (TEMS) procedure. The retractor **7100** comprises an inner or distal ring **7110,** an outer or proximal ring **7120,** a tubular body **7130,** and a funnel segment **7140** extending between and coupling the inner ring **7110** and the outer ring **7120.** The tubular body **7130** comprises a relatively flexible material such as a KRATON® material or a silicone rubber material, which is substantially cylindrical in the illustrated retractor. In other retractors, the tubular body **7130** has another shape, for example, an oval cross section. Some examples of the tubular body **7130** comprise one or more coatings that provide additional functionality, for example, an anti-microbial coating.

In the illustrated retractor, the inner ring **7110** is substantially flush with a distal end of the tubular body **7130** such that the retractor **7100** has a generally tubular configuration extending distally of the funnel segment **7140** to the distal end. Exampless of the inner ring **7110** are sufficiently flexible and compliant to be compressed and/or deformed for insertion into a body orifice such as a patient's anus during a transanal surgical procedure. When subsequently released within an associated body cavity, the inner ring **7110** substantially returns to its original shape or footprint. In some retractors, the inner ring **7110** assumes a substantially circular shape substantially flush with the generally cylindrical tubular body **7130** in a relaxed state, for example, when released within a body cavity. In other retractors, the inner ring **7110** has another shape in the relaxed state, for example, an oval. The inner ring **7110** assumes a different shape when compressed for insertion through an incision or body orifice, for example, a substantially oval shape, a generally linear shape, a tear-drop shape, or another suitable shape. In other retractors, the inner ring **7110** is substantially rigid, that is, non-compliant under the ordinary conditions under which it is used.

With continued reference to FIGS. **6D-6F****,** in some retractors, the inner ring **7110** can be shaped and configured to facilitate insertion through a natural orifice. For example, the inner ring **7110** can include a radiused edge to facilitate atraumatic entry through a natural orifice. In other retractors, the inner ring **7110** can include a beveled edge to facilitate entry through a natural orifice. Furthermore, in the illustrated retractor, the inner ring **7110** can be formed at an angle transverse to a longitudinal axis defined by the tubular body **7130.** Advantageously, such an angled inner ring **7110** can facilitate insertion of the retractor **7100** through a natural orifice. In other retractors, the inner ring **7110** can be substantially perpendicular to the longitudinal axis defined by the tubular body.

With continued reference to FIGS. **6D-6F****,** the outer ring **7120** is proximal the funnel section **7140.** In the illustrated retractor, the outer ring **7120** has a substantially circular footprint. As further discussed herein, the outer ring **7120** can be sized and configured to sealingly couple to a cap or other access device thereon. In some retractors, as discussed above with reference to the embodiments of FIGS. **6A-6C**, one or more suture points can be disposed on the retractor **7100** adjacent the outer ring **7120.**

With continued reference to FIGS. **6D-6F****,** the tubular body **7130** can have a generally circular profile defining a generally cylindrical passage **7150.** The generally cylindrical passage **7150** is desirably large enough to accommodate more than one laparoscopic instrument there through such that a single natural orifice access device can be used to provide access for multiple surgical instruments in a body cavity. Moreover, generally cylindrical passage **7150** is desirably large enough such that multiple surgical instruments positioned there through can be translated or pivoted relative to one another, allowing a surgeon to manipulate the instruments as desired during a surgical procedure. The generally cylindrical passage extends between a proximal end **7152** of the retractor **7100** adjacent the outer ring **7120** to a distal end **7154** of the retractor **7100** adjacent the inner ring **7110** (FIG. **6D**).

With reference to FIG. **6D**, the funnel segment **7140** provides a diametric reduction between the relatively large diameter of the outer ring **7120,** which is sized and configured to be removably coupled to an access device such as a cap, and the relatively smaller diameter of the passage **7150,** which is sized to fit within a natural orifice with minimal distention of the orifice. The funnel segment **7140** has an inner surface **7142** which can provide a bearing surface for an obturator used to advance to the retractor **7100** into a body cavity. In some retractors, the funnel segment **7140** can have a substantially linear taper between the relatively large diameter and the relatively smaller diameter such that the inner surface **7142** is a frusto-conical segment. In other retractors, the funnel segment **7140** can have a curved profile between the relatively large diameter and the relatively smaller diameter.

A natural orifice access system can include a retractor **7100** and an obturator, such as described above with reference to FIG. **6G**. The obturator can have a proximal bearing surface **6410** sized and configured to bear against the inner surface **7142** of the funnel segment **7140** and a distal dilation surface **6420** sized and configured to expand a natural orifice for passage of the retractor **7100.** Thus, during insertion of the retractor **7100** into a natural orifice, the dilation surface expands a pathway to a surgical site in a body cavity while the obturator bears on the inner surface **7142** of the funnel segment **7140** to advance the retractor **7100** into position in the surgical site. Furthermore, the obturator can have a handle **6430** at a proximal end thereof adapted to facilitate selective twisting or rotation of the obturator about a longitudinal axis thereof during insertion.

In an alternative example, shown in FIG. **6I**, the obturator **6405** includes a straight shaft piece **6425** between the distal dilation surface **6420** and the proximal bearing surface **6410** that facilitates dilation of the natural orifice prior to inserting the retractor. It can then be combined with the retractor **7100** to help ease insertion, as shown in FIG. **6J**.

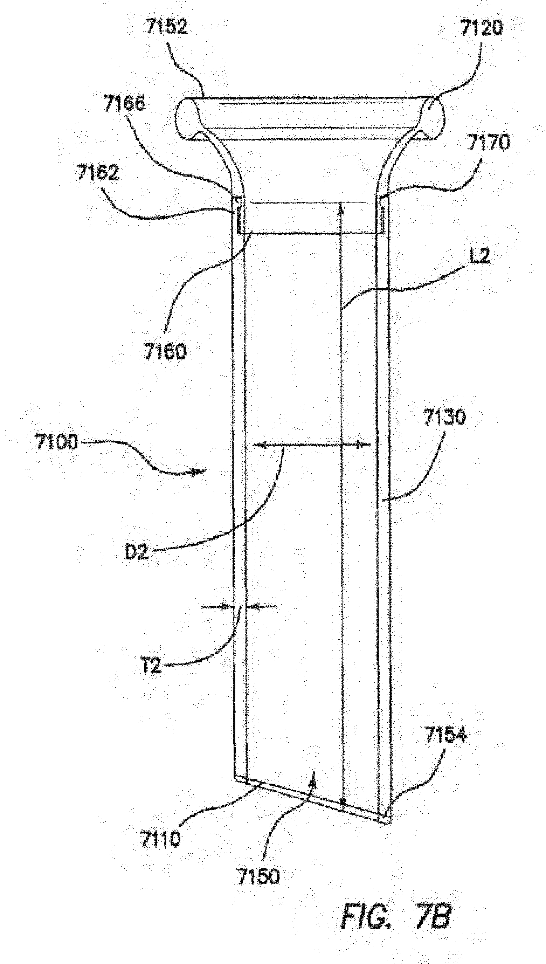

With reference to FIG. **7B**, it can be desirable that the outer ring **7120** is relatively stiff compared with the relatively flexible tubular body **7130** of the retractor **7100** so that the outer ring **7120** can sealingly engage an access device such as a cap. In the illustrated example, the retractor **7100** is formed in a multiple-shot molding process. For example, an inner segment of the retractor **7100** defined by the tubular body **7130** and the inner ring **7110** is formed in one molding operation from a flexible material, and an outer segment of the retractor **7100** defined by the funnel segment **7140** and the outer ring **7120** is formed in another molding operation from a relatively rigid material such as a polycarbonate material or other suitable material.

Alternatively, a multiple-shot molding process can be varied such that the resulting inner and outer segments are different from those of the illustrated example. For example, the inner segment can include the tubular body **7130**, the inner ring **7110**, and a portion of the funnel segment **7140**, while the outer segment can include a portion of the funnel segment **7140** and the outer ring **7120**. In certain other examples, the inner segment can include the inner ring **7110** and a portion of the tubular body **7130**, while the outer segment can include a portion of the tubular body **7130**, the funnel segment **7140**, and the outer ring **7120**.

With reference to FIGS. **6D** and **7B**, a retractor **7100** formed in a multiple-shot molding process can include one or more retention members **7160** on the inner segment and the outer segment to maintain the position of the inner segment relative to the outer segment. For example, a distal end of the outer segment can include one or more protrusions **7162** extending radially outwardly from the funnel segment **7140** and one or more recesses **7164** recessed radially inwardly from the funnel segment **7140** at an interface region of the inner segment and the outer segment of the retractor **7100**. In the illustrated example, the distal end of the outer segment includes a plurality of protrusions **7162** alternating with a plurality of recesses **7164** there between. Moreover, in some examples, the outer segment can include an annular groove **7170** formed in the funnel segment **7140** at an interface region of the inner segment and the outer segment of the retractor **7100**. The inner segment of the retractor **7100** can include an annular member **7166** disposed within and matingly engaging the groove **7170** to maintain the position of the inner segment relative to the outer segment.

With reference to FIG. **7B**, a cross-sectional view of retractor **7100** is shown. In the illustrated retractor, the tubular body **7130** is formed of a flexible material having a predetermined fixed length L2, inner diameter D2, and wall thickness T2. The fixed length L2, inner diameter D2, and wall thickness T2 are selected to accommodate the anatomy of a natural orifice, such as the anal orifice of a majority of patients. It is contemplated that the retractor **7100** can be scaled to different sizes for patients of different ages. Furthermore, in some examples, the retractor can include a telescopic tubular body such that the tubular body can be selectively positioned at a variety of lengths depending on patient anatomy and the location of the surgical site within the body cavity. Desirably, the wall thickness T2 and material of the tubular body **7130** are selected such that the tubular body **7130** is resilient enough to maintain the passage **7150** there through when positioned in the natural orifice. Moreover, desirably, the inner diameter, D2 is sufficiently large to accommodate multiple surgical instruments. For example, in examples of the retractor **7100** adapted for use in a transanal surgical procedure, the inner diameter D2 and thickness T2 can be sized such that an outer diameter of the retractor can be between approximately 30 mm and 70 mm, desirably between approximately 35 mm and 50 mm, and in one embodiment approximately 40 mm. Additionally, desirably, the fixed length L2 is sufficiently long such that the inner ring **7110** can be positioned at a surgical site within a body cavity and the outer ring **7120** can be positioned outside the natural orifice. In some retractors, the fixed length L2 is of a length such that the device has an overall length between the proximal end **7152** and the distal end **7154** of between approximately 100 mm and approximately 200 mm, desirably between approximately 120 mm and 180 mm, more desirably between approximately 140 mm and 160 mm, and in one embodiment, approximately 150 mm.

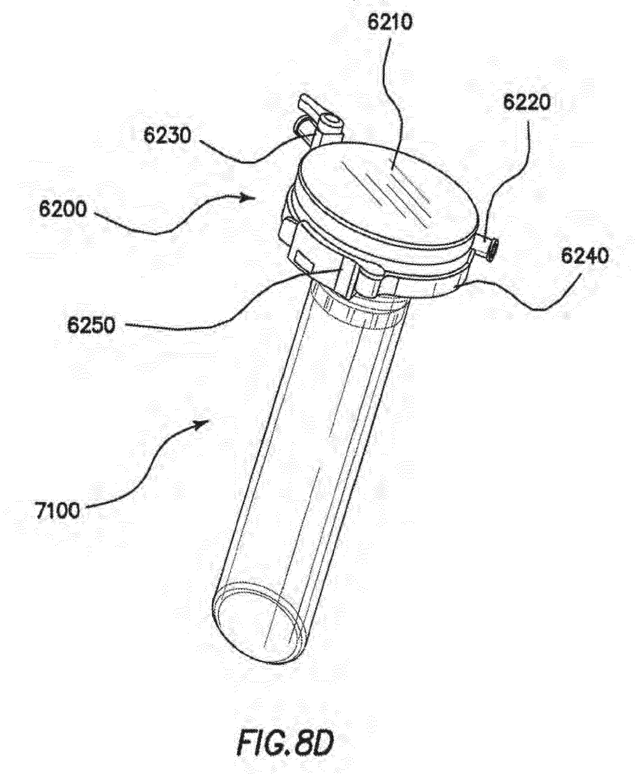

With reference to FIG. **8D**, a perspective view of a natural orifice access device having a cap **6200** substantially similar to that described with respect to FIGS. **8A-8C** removably coupled to a retractor **7100** is illustrated. In the illustrated device, the cap **6200** comprises a sealable access surface **6210** such as a gel pad surface as described in further detail herein. In certain devices, the cap **6200** can also comprise at least one gas or fluid port **6220, 6230.** In the illustrated device, the cap **6200** comprises two gas or fluid ports **6220, 6230,** such that one port can be used for gas insufflation and the other port can be used for ventilation for example when electrosurgery is performed through the access device. In certain devices, at least one of the gas or fluid ports **6220, 6230** comprises a valve such as a stopcock valve to selectively control the flow of fluid there through.

With continued reference to FIG. **8D**, a top view of the natural orifice access device is illustrated. The sealable access surface **6210** can be encircled by and restrained by an annular frame **6240** such as a split ring having a clamp **6250.** The clamp **6250** can be movable between an open configuration in which the cap **6200** is selectively removable from the retractor **7100** and a clamped configuration in which the cap **6200** can be secured to the retractor **7100.** For example, the annular frame **6240** can be positioned peripherally around the outer ring **7120** with the clamp **6250** in the open configuration and the clamp moved to the clamped configuration to sealingly fix the cap **6200** to the retractor **7100.** Accordingly, the cap **6200** can be easily removed during a surgical procedure to facilitate removal of excised tissue from a surgical site through the retractor **7100.**

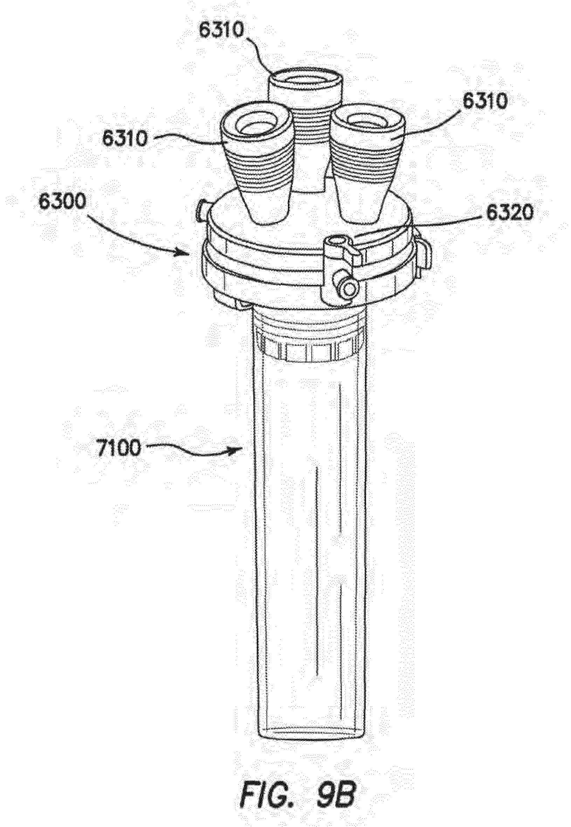

With reference to FIG. **9B**, another example of a natural orifice access device is illustrated, which can include a cap **6300** substantially similar to that described above with reference to FIG. **9A** removably coupled to a retractor **7100** such as that described above with respect to FIGS. **6D-F**, **7B**, and **8D**. The cap **6300** can include multiple trocar access devices **6310** positioned through an access surface **6320** thereof. Advantageously, the multiple trocar access devices **6310** allow for easy placement and manipulation of multiple laparoscopic instruments in a surgical site through a single natural orifice.

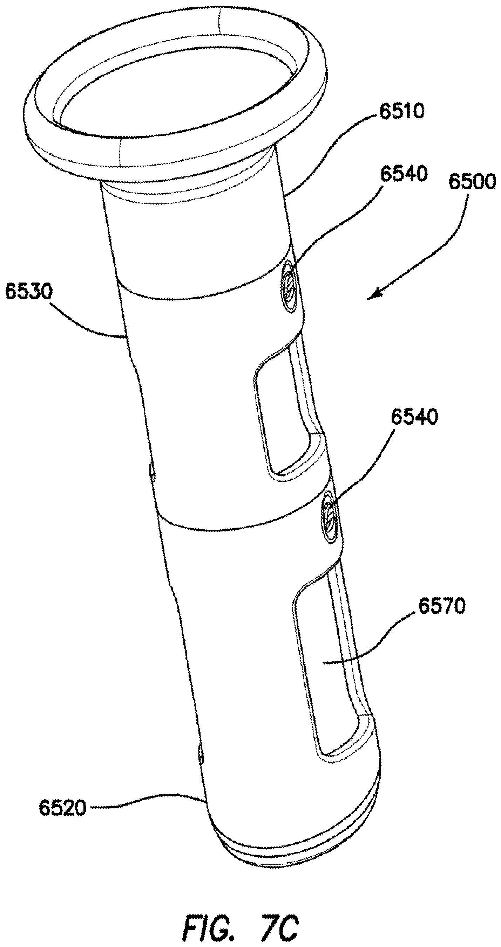

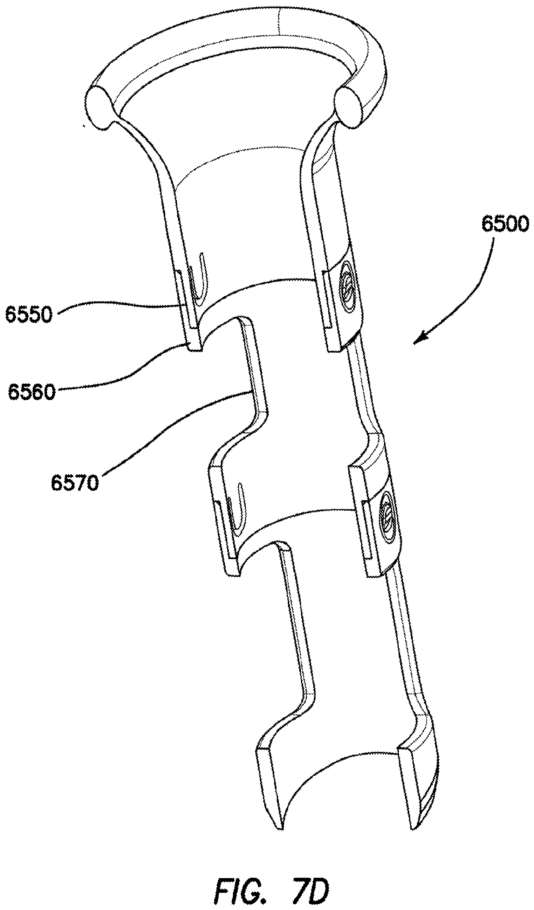

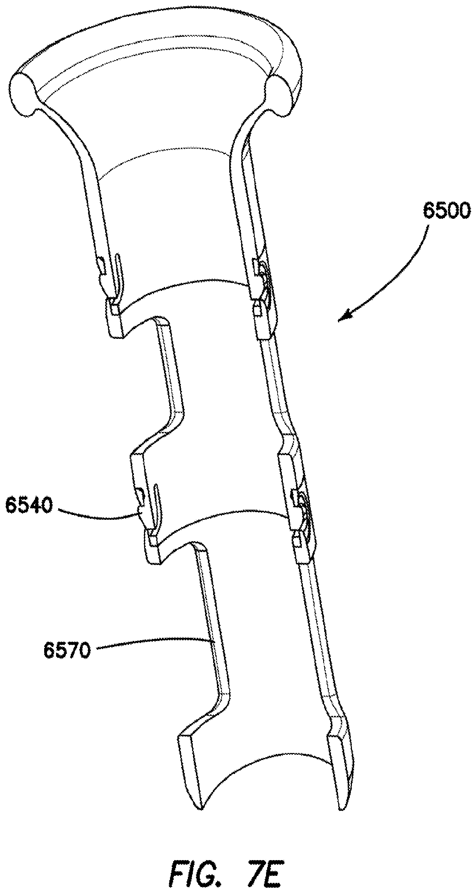

As discussed herein, the retractors shown in FIGs. **7A** and **7B** can include a telescopic tubular body such that the tubular body can be selectively positioned at a variety of lengths depending on patient anatomy and the location of the surgical site within the body cavity. In another example, illustrated in FIG. **7C**, the tubular body may be formed in sections of varying length that slidingly engage and snap lock together to provide a variety of lengths, depending of the number and size of the sections selected and assembled. With reference to FIG. **7C**, a perspective view of a retractor **6500** is shown having three sections: an outer ring section **6510,** an inner ring section **6520,** and an intermediate section **6530** disposed between the other two sections. The three sections are held together by a snap lock mechanism **6540.** Each section terminates at the distal end with an annular groove **6550** that slidingly engages with the proximal end **6560** of the next section, best shown in the cross section view of FIG. **7D**. The snap lock mechanism is shown in cross-section in FIG. **7E**. The tubular body of the embodiment shown in FIG. **7C-E** is preferably formed from a relatively stiff material, such as a polycarbonate.

Optionally, as shown in FIG. **7C-7F**, the tubular body of the retractor can include cut-out portions or windows **6570,** to provide access to regions of the anatomy that would otherwise be obscured by the tubular body while the retractor is in place. Thus, the retractor can be inserted into the body orifice or incision to provide retraction and to protect the lining of the body cavity, and then manipulated to align the window(s) to the sites of interest in the body cavity for access by surgical instruments.

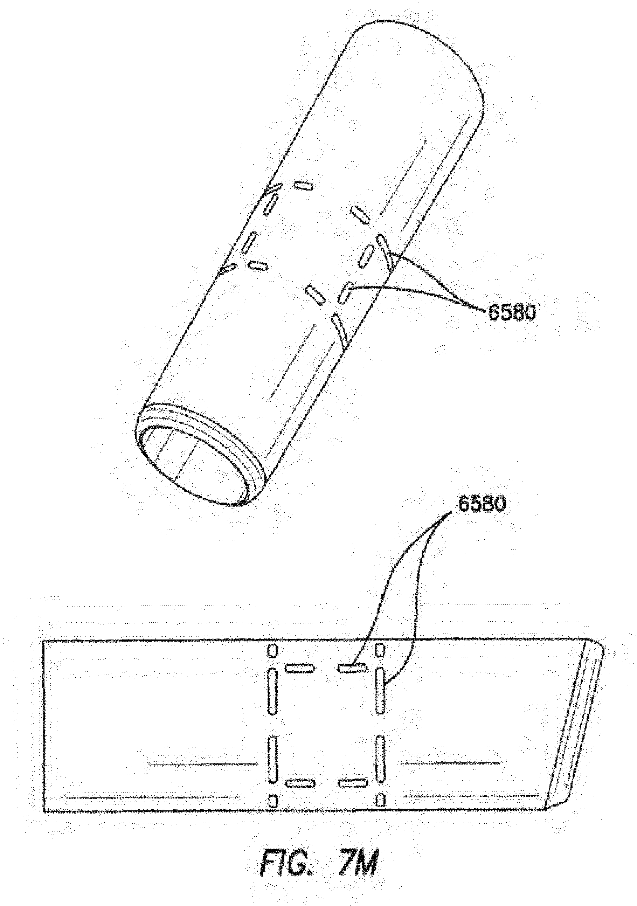

As will be appreciated, such cut-out portions may be provided in retractors having tubular bodies of both rigid and flexible construction, as well as tubular bodies formed as a single piece or in sections. FIG. **7M** shows an example of a flexible tubular body of a retractor, both in side view and in perspective view, wherein the tubular body contains perforations **6580.** The tubular body can be cut or torn at the perforations to vary the length of the tubular body and/or to incorporate cut-out portions into the tubular body. The tubular body of the embodiment shown in FIG. **7M** is preferably formed from a relatively flexible material, such as KRATON® or PELLETHANE®.

In the illustrated natural orifice access systems of FIGs. **9A** and **9B**, the trocar access devices **6310** have a relatively low profile, that is, protrude minimally above the access surface **6320** and/or below the distal surface of the cap **6300.** Accordingly, the trocar access devices **6310** are shorter than a length of a typical trocar and comprise a seal assembly positioned above the access surface **6320** and a cannula extending through the gel pad of the cap **6300.** The reduced length of the trocar access devices **6310** allows increased angular or pivotal motion for instruments extending there through, and also permits the use of curved and/or angled instruments.

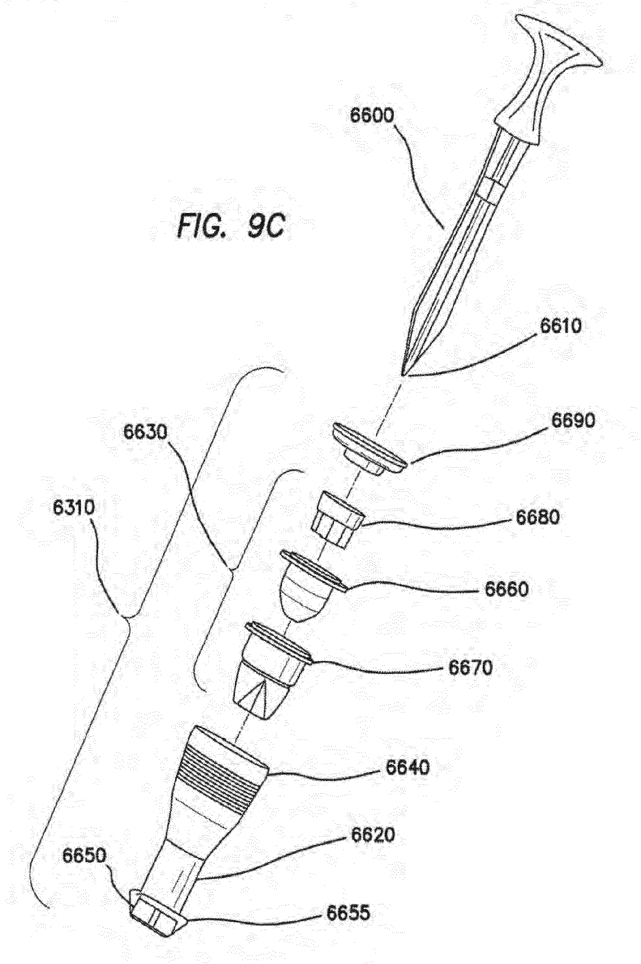

FIG. **9C** is an exploded view of an example of a trocar access device **6310** and obturator **6600,** which is a component of some examples of the access device system. In the illustrated device, the obturator **6600** comprises a pointed, puncture tip **6610.**

The trocar access device **6310** comprises a proximal end, a distal end, and a longitudinal axis. The trocar access device **6310** comprises a cannula **6620** extending along the longitudinal axis. A trocar seal **6630** is disposed at the proximal end of the cannula **6620,** contained within a housing **6640.** A retainer **6650** is disposed at the distal end or tip of the cannula **6620.**

The cannula **6620** comprises a tubular body dimensioned to accommodate an instrument or instruments received there through. In the illustrated example, the cannula **6620** is a substantially cylindrical tube, and extends through the cap **6300** in use. In the illustrated example, the cannula **6620** is comparatively short because the cannula need only traverse the cap 6300 (FIG. **9A-B**), which has a known and consistent thickness, rather than a body wall. Accordingly, some examples of the cannula **6620** are not more than about 2-times longer, about 1.5-times longer, about 1.2-times longer, or about 1.1-times longer than the thickness of the gel pad. In some examples, the cannula **6620** is less than about 20 mm, about 10 mm, or about 5 mm longer than the thickness of the gel pad. In some examples, the cannula **6620** is about as long as the gel pad is thick. In other examples, the cannula **6620** has a different length, for example, a length typical for a cannula used for traversing a body wall. Shorter length cannula permit increased angular degrees of freedom for instruments passing there through. Examples of shorter cannula also accommodate curved instruments. The cannula **6620** comprises any suitable biocompatible material. In some examples, the cannula **6620** comprises a flexible material.

The illustrated trocar seal **6630** comprises an instrument or septum seal **6660** and a zero seal **6670.** Optionally, a shield **6680** may be disposed within the instrument seal **6660.** The instrument seal **6660** seals instruments passing there through, thereby maintaining pressurization in a body cavity such as pneumoperitoneum or pneumorectum. The zero seal **6670** provides a seal when no instrument passes through the trocar seal **6630.** The instrument seal **6660** and zero seal **6670** are received in a housing **6640** disposed at the proximal end of the cannula **6620** and secured therein by a seal cover **6690.**

The retainer **6650** is disposed at or near the distal end of the cannula **6620.** In some examples, the retainer **6650** and cannula **6630** are integrated, while in other examples, the retainer **6650** and cannula **6630** are not integrated. In the illustrated example, the proximal end of the retainer **6650** comprises a flange **6655** that is generally flat and perpendicular to the longitudinal axis, while the distal end is tapered, narrowing toward the distal end of the cannula **6620.** The flange **6655** reduces the likelihood of accidental or inadvertent removal of the trocar access device **6310** from the cap. Some examples of the proximal face of the flange **6655** comprise additional anchoring features, for example, at least one of barbs, spikes, ridges, texturing, and the like, which are configured to penetrate or bite into a distal face of the cap **6300**. In some examples, a diameter of the flange **6655** is from about 1.2 to about 2.5 times wider, or from about 1.5 to about 2.0 times wider than an outer diameter of the cannula **6630**. Some examples of the trocar access device **6310** are 5-mm trocars, in which the outer diameter of the cannula **6620** is from about 7 mm to about 8 mm.

The tapered end of the retainer **6650** facilitates insertion of the trocar access device **6310** through the cap, either by itself, or when assembled with the obturator 6600 extending there through. For example, in some devices, the retainer **6650** is inserted through a preformed opening in the cap **6300**.

In some examples in which the retainer **6650** and cannula **6620** are not integrated, that is, are separate components, the retainer **6650** is secured to the cannula **6620** after the cannula **6620** is inserted through the cap. In some examples, the cannula **6620** and retainer **6650** are secured mechanically, for example, using latches, screw threads, clips, lock rings, ratchets, and the like. In some examples, the cannula **6620** and retainer **6650** are secured adhesively. In some examples, the position of the retainer **6650** is adjustable, for example, to accommodate caps of different thicknesses. In some examples, the cannula **6620** and/or retainer **6650** is secured to the cap, for example, adhesively.

An example of a procedure for retracting a body orifice is described with reference to the examples of the retractor **6100** illustrated in FIGS. **6A-6C**, **7A**, **8A-8C**, and **9A**, and the examples of retractor **7100** illustrated in FIGS. **6D-6F**, **7B**, **8D**, and **9B**. In use, the surgical wound retractor **6100, 7100** is inserted into a body orifice, such as the vagina (FIG. **2**), mouth (FIG. **3**) or anus (FIG. **4**). The inner ring **6110, 7110** is folded or compressed into an oval or other suitable shape and urged through the incision or body orifice into an associated body cavity. Once the inner ring **6110, 7110** is fully disposed within the associated body cavity, it is allowed to resume its original, relaxed shape, for example, substantially circular, oval, or other original shape. In some embodiments, the inner ring **6110** is then pulled upward against the inner surface of the body cavity, for example, by pulling the outer ring **6120** upward. An outer surface of the tubular body **6130, 7130** retracts the natural orifice.

As illustrated in FIG. **5**, some examples of the access device **5000** comprise a cap, cover, or lid **5500** coupled to the outer ring of the retractor **5100,** which seals the retractor **5100,** for example, for maintaining pressurization within a body cavity such as pneumoperitoneum or pneumorectum. In some examples, lid **5500** is removable, for example to provide access into the body cavity. Some examples of the lid **5500** comprise a transparent or translucent portion, thereby allowing a user to view into the body cavity without removing the lid **5500.** As will be described below, one example of a lid **5500** is a gel cap. In some examples, a cross-sectional shape of the outer ring **6120** (FIG. **6A**), **7120** (FIG. **6D**) of the retractor is selected to reduce or prevent the lid **5500** from partial and/or incorrect coupling to the outer ring **6110** (FIG. **6A**), **7120** (FIG. **6D**) of the wound retractor. Such cross-sectional shapes include oval and rectangular, or any other suitable cross-sectional shape that provides the desired functionality, for example, hexagonal, octagonal, and the like. Additionally, depending on the use and on surgeon preference, in some examples, each of the inner ring **6110, 7110** and outer ring **6120, 7120** of the wound retractor includes independently variable design configurations. For example, examples of the inner ring **6110, 7110** and/or the outer ring **6120, 7120** are rigid or flexible, and have footprints, cross-sectional shapes, and/or dimensions dependent on the intended use, for example, circular or oval footprints, diameters dependent on incision or orifice dimensions, or cross-sectional dimensions dependent on retraction force. In some examples, the inner ring **6100** may extend radially out from the tubular body **6130** when deployed, stabilizing the retractor within the body orifice (FIG. **7A**). In other examples, the inner ring **7110** may be flush with the tubular body **7130,** as where, for example, the length L2 of the tubular body is sufficient to stabilize the retractor within the body orifice (FIG. **7B**).

While certain embodiments have been particularly shown and described with reference to exemplary embodiments thereof, it will be understood by those of ordinary skill in the art that various changes in form and details may be made therein without departing from the scope of the invention as defined by the following claims.

## Claims

1. A surgical access port system adapted for performing laparoscopic surgical procedures at a natural orifice comprising a retractor (6100) comprising:
an outer ring (6120), wherein the outer ring (6120) is configured to be disposed proximate the natural orifice of the patient and substantially surround the orifice;
a tubular body (6130), wherein the tubular body (6130) defines a generally cylindrical passage large enough to accommodate at least one laparoscopic instrument there through;
a funnel segment (6140) extending between and coupling the outer ring (6120) and the tubular body (6130), wherein the funnel segment (6140) provides a diametric reduction between the relatively large diameter of the outer ring (6120) and the relatively smaller diameter of the tubular body (6130), which is sized to fit within a natural orifice with minimal distention of the orifice;
an inflation port (6134) disposed within the funnel segment (6140);
an inflatable member (6132) disposed on a distal edge of the tubular body (6130), wherein the inflatable member (6132) is connected to the inflation port (6134) by a channel (6136); and
an obturator (6400) comprising a proximal bearing surface (6410) sized and configured to bear against an inner surface (6142) of the funnel segment (6140), and a distal dilation surface (6420) sized and configured to expand the natural orifice;
**characterized in that** the obturator (6400) further comprises an indent (6402) disposed along the periphery of the proximal bearing surface (6410) sized and configured to provide clearance for the inflation port (6134).

2. The surgical access port system of claim 1, wherein the tubular body (6130) comprises two or more interlocking sections.

3. The surgical access port system of claim 1 or 2, wherein the tubular body (6130) comprises at least one window (6570).

4. The surgical access port system of claim 1, 2 or 3, wherein the funnel segment (6140) comprises an inner surface (6142) that can provide a bearing surface for an obturator (6400) used to advance to the retractor (6100) into a body cavity.

5. The surgical access port system of any preceding claim, wherein the funnel segment (6140) has a substantially linear taper between the relatively large diameter and the relatively smaller diameter.

6. The surgical access port system of any one of claims 1 to 4, wherein the funnel segment (6140) has a curved profile between the relatively large diameter and the relatively smaller diameter.

7. The surgical access port system of any preceding claim, wherein the tubular body (6130) is formed from a relatively flexible material and the funnel segment (6140) and the outer ring (6120) are formed from a relatively rigid material.

8. The surgical access port system of any preceding claim, wherein the tubular body (6130) comprises a plurality of perforations (6580).

9. The surgical access port system of claim 8, wherein the perforations (6580) are arranged so that the tubular body (6130) may be cut or torn at the perforations (6580) to shorten the tubular body (6130).

10. The surgical access port system of claim 8, wherein the perforations (6580) are arranged so that the tubular body (6130) may be cut or torn at the perforations (6580) to incorporate cut out portions in the tubular body (6130).

11. The surgical access port system of claim 8 or 9, wherein the perforations (6580) are located in a central portion of the tubular body (6130).

12. The surgical access port system of claims 9 and 10, wherein at least some of the perforations (6580) are arranged to be cut or torn to either shorten the length of the tubular body (6130) or to incorporate cut out portions in the tubular body (6130), as desired.

13. The surgical access port system of claim 1, further comprising a removable cap (6200), wherein the cap (6200) is adapted to sealingly engage the outer ring (6120).

14. The surgical access port system of claim 13, wherein the cap (6200) comprises a sealable access surface (6210).

## Patentansprüche

1. Ein chirurgisches Zugangsanschlusssystem, das zum Durchführen laparoskopischer chirurgischer Eingriffe an einer natürlichen Öffnung angepasst ist, das einen Retraktor (6100) beinhaltet, der Folgendes beinhaltet:
einen Außenring (6120), wobei der Außenring (6120) dazu ausgelegt ist, in der Nähe der natürlichen Öffnung des Patienten angeordnet zu sein und die Öffnung im Wesentlichen zu umgeben;
einen röhrenförmigen Körper (6130), wobei der röhrenförmige Körper (6130) einen im Allgemeinen zylindrischen Durchgang definiert, der groß genug ist, um mindestens ein laparoskopisches Instrument durch diesen aufzunehmen;
ein Trichtersegment (6140), das sich zwischen dem Außenring (6120) und dem röhrenförmigen Körper (6130) erstreckt und diese koppelt, wobei das Trichtersegment (6140) eine Durchmesserverkleinerung zwischen dem relativ großen Durchmesser des Außenrings (6120) und dem relativ kleineren Durchmesser des röhrenförmigen Körpers (6130) bereitstellt, der so dimensioniert ist, dass er in eine natürliche Öffnung hineinpasst, wobei die Öffnung minimal aufgeweitet wird;
einen Aufblasanschluss (6134), der innerhalb des Trichtersegments (6140) angeordnet ist;
ein aufblasbares Element (6132), das auf einem distalen Rand des röhrenförmigen Körpers (6130) angeordnet ist, wobei das aufblasbare Element (6132) durch einen Kanal (6136) mit dem Aufblasanschluss (6134) verbunden ist; und
einen Obturator (6400), der Folgendes beinhaltet: eine proximale Auflagefläche (6410), die so dimensioniert und ausgelegt ist, dass sie an einer Innenfläche (6142) des Trichtersegments (6140) aufliegt, und eine distale Dilatationsfläche (6420), die so dimensioniert und ausgelegt ist, dass sie die natürliche Öffnung aufweitet;
**dadurch gekennzeichnet, dass** der Obturator (6400) ferner einen Einschnitt (6402) beinhaltet, der entlang des Umfangs der proximalen Auflagefläche (6410) angeordnet ist, der so dimensioniert und ausgelegt ist, dass er einen freien Raum für den Aufblasanschluss (6134) bereitstellt.

2. Das chirurgische Zugangsanschlusssystem gemäß Anspruch 1, wobei der röhrenförmige Körper (6130) zwei oder mehr ineinandergreifende Abschnitte beinhaltet.

3. Das chirurgische Zugangsanschlusssystem gemäß Anspruch 1 oder 2, wobei der röhrenförmige Körper (6130) mindestens ein Fenster (6570) beinhaltet.

4. Das chirurgische Zugangsanschlusssystem gemäß Anspruch 1, 2 oder 3, wobei das Trichtersegment (6140) eine Innenfläche (6142) beinhaltet, die eine Auflagefläche für einen Obturator (6400) bereitstellen kann, der verwendet wird, um den Retraktor (6100) in eine Körperhöhle vorzuschieben.

5. Das chirurgische Zugangsanschlusssystem gemäß einem der vorhergehenden Ansprüche, wobei das Trichtersegment (6140) eine im Wesentlichen lineare Verjüngung zwischen dem relativ großen Durchmesser und dem relativ kleineren Durchmesser aufweist.

6. Das chirurgische Zugangsanschlusssystem gemäß einem der Ansprüche 1 bis 4, wobei das Trichtersegment (6140) ein gebogenes Profil zwischen dem relativ großen Durchmesser und dem relativ kleineren Durchmesser aufweist.

7. Das chirurgische Zugangsanschlusssystem gemäß einem der vorhergehenden Ansprüche, wobei der röhrenförmige Körper (6130) aus einem relativ flexiblen Material gebildet ist und das Trichtersegment (6140) und der Außenring (6120) aus einem relativ starren Material gebildet sind.

8. Das chirurgische Zugangsanschlusssystem gemäß einem der vorhergehenden Ansprüche, wobei der röhrenförmige Körper (6130) eine Vielzahl von Perforationen (6580) beinhaltet.

9. Das chirurgische Zugangsanschlusssystem gemäß Anspruch 8, wobei die Perforationen (6580) so angeordnet sind, dass der röhrenförmige Körper (6130) an den Perforationen (6580) abgeschnitten oder abgerissen werden kann, um den röhrenförmigen Körper (6130) zu verkürzen.

10. Das chirurgische Zugangsanschlusssystem gemäß Anspruch 8, wobei die Perforationen (6580) so angeordnet sind, dass der röhrenförmige Körper (6130) an den Perforationen (6580) abgeschnitten oder abgerissen werden kann, um ausgeschnittene Anteile in den röhrenförmigen Körper (6130) einzugliedern.

11. Das chirurgische Zugangsanschlusssystem gemäß Anspruch 8 oder 9, wobei die Perforationen (6580) sich in einem zentralen Anteil des röhrenförmigen Körpers (6130) befinden.

12. Das chirurgische Zugangsanschlusssystem gemäß Ansprüchen 9 und 10, wobei mindestens einige der Perforationen (6580) so angeordnet sind, dass sie abgeschnitten oder abgerissen werden können, um je nach Wunsch entweder die Länge des röhrenförmigen Körpers (6130) zu verkürzen oder ausgeschnittene Anteile in den röhrenförmigen Körper (6130) einzugliedern.

13. Das chirurgische Zugangsanschlusssystem gemäß Anspruch 1, das ferner einen entfernbaren Deckel (6200) beinhaltet, wobei der Deckel (6200) zum abdichtenden Eingriff mit dem Außenring (6120) angepasst ist.

14. Das chirurgische Zugangsanschlusssystem gemäß Anspruch 13, wobei der Deckel (6200) eine abdichtbare Zugangsfläche (6210) beinhaltet.

## Revendications

1. Un système de port d'accès chirurgical adapté à l'exécution d'interventions chirurgicales laparoscopiques au niveau d'un orifice naturel comprenant un rétracteur (6100) comprenant :
Un anneau extérieur (6120), cet anneau extérieur (6120) étant configuré pour être placé au niveau de l'orifice naturel du patient et substantiellement autour de cet orifice ;
Un corps tubulaire (6130), ce corps tubulaire (6130) définissant un passage généralement cylindrique suffisamment large pour y faire passer au moins un instrument de laparoscopie :
Un segment en forme d'entonnoir (6140) s'étendant entre et raccordé à l'anneau extérieur (6120) et le corps tubulaire (6130), ce segment en forme d'entonnoir (6140) fournissant une réduction diamétrale entre le diamètre relativement large de l'anneau extérieur (6120) et le diamètre relativement moindre du corps tubulaire (6130), qui est d'une taille permettant son insertion dans l'orifice naturel avec une dilatation minimum de cet orifice ;
Un port de gonflage (6134) disposé au sein du segment en forme d'entonnoir (6140) ;
Un membre gonflable (6132) disposé sur un bord distal du corps tubulaire (6130), ce membre gonflable (6132) étant connecté au port de gonflage (6134) par un conduit (6136) ; et
Un obturateur (6400) comprenant une surface d'appui proximale (6410) d'une taille et d'une configuration permettant de s'appuyer contre une surface intérieure (6142) du segment en forme d'entonnoir et (6140) et une surface de dilatation distale (6420) d'une taille et d'une configuration permettant de dilater l'orifice naturel ;
**caractérisé en ce que** l'obturateur (6400) comprend en outre une encoche (6402) disposée le long de la périphérie de la surface d'appui proximale (6410) d'une taille et d'une configuration permettant assez d'espace pour le port de gonflage (6134).

2. Le système de port d'accès chirurgical de la revendication 1, dans lequel le corps tubulaire (6130) se compose d'au moins deux sections imbriquées.

3. Le système de port d'accès chirurgical de la revendication 1 ou 2, dans lequel le corps tubulaire (6130) comprend au moins une fenêtre (6570).

4. Le système de port d'accès chirurgical de la revendication 1, 2 ou 3, dans lequel le segment en forme d'entonnoir (6140) comprend une surface intérieure (6142) qui peut fournir une surface d'appui à un obturateur (6400) utilisé pour avancer vers le rétracteur (6100) dans une cavité du corps.

5. Le système de port d'accès chirurgical de l'une quelconque des revendications précédentes, dans lequel le segment en forme d'entonnoir (6140) est d'une forme substantiellement évasée entre le diamètre relativement large et le diamètre relativement moindre.

6. Le système de port d'accès chirurgical de l'une quelconque des revendications 1 à 4, dans lequel le segment en forme d'entonnoir (6140) est doté d'un profil courbe entre le diamètre relativement large et le diamètre relativement moindre.

7. Le système de port d'accès chirurgical de l'une quelconque des revendications précédentes, dans lequel le corps tubulaire (6130) est formé dans un matériau relativement flexible et le segment en forme d'entonnoir (6140) et l'anneau extérieur (6120) sont formés dans un matériau relativement rigide.

8. Le système de port d'accès chirurgical de l'une quelconque des revendications précédentes, dans lequel le corps tubulaire (6130) comprend une pluralité de perforations (6580).

9. Le système de port d'accès chirurgical de la revendication 8, dans lequel les perforations (6580) sont disposées de manière à ce que le corps tubulaire (6130) puisse être coupé ou déchiré au niveau des perforations (6580) de manière à raccourcir le corps tubulaire (6130).

10. Le système de port d'accès chirurgical de la revendication 8, dans lequel les perforations (6580) sont disposées de manière à ce que le corps tubulaire (6130) puisse être coupé ou déchiré au niveau des perforations (6580) de manière à incorporer des portions coupées dans le corps tubulaire (6130).

11. Le système de port d'accès chirurgical de la revendication 8 ou 9, dans lequel les perforations (6580) sont situées dans une partie centrale du corps tubulaire (6130).

12. Le système de port d'accès chirurgical des revendications 9 et 10, dans lequel au moins certaines des perforations (6580) sont disposées pour être coupées ou déchirées afin soit de raccourcir la longueur du corps tubulaire (6130) soit d'incorporer des portions coupées dans le corps tubulaire (6130), selon le besoin.

13. Le système de port d'accès chirurgical de la revendication 1, comprenant en outre un capuchon amovible (6200), ce capuchon amovible (6200) étant adapté pour s'enclencher dans l'anneau extérieur (6120) en formant un joint étanche.

14. Le système de port d'accès chirurgical de la revendication 13, dans lequel le capuchon (6200) comprend une surface d'accès formant un joint étanche (6210).
